# EUROPÄISCHE PATENTSCHRIFT

(11) **EP 2 289 483 B1**
(45) Veröffentlichungstag und Bekanntmachung des Hinweises auf die Patenterteilung: **08.03.2017**
(21) Anmeldenummer: 09168604.8
(22) Anmeldetag: 25.08.2009
(51) Int. Cl.: A61K 6/083, C07D 237/08, C07D 259/00, C07D 323/00, C07D 327/02, C07D 498/08

(54) **Verwendung von polymerisierbaren makrocyclischen Polyethern und makrocyclischen heteroanalogen Polyethern in Dentalmaterialien**
Use of polymerisable macrocyclic polyethers and macrocyclic heteroanalogous polyethers in dental materials
Utilisation de polyéthers macrocycliques polymérisables et de polyéthers macrocycliques hétéroanalogues dans des matériaux dentaires

(43) Veröffentlichungstag der Anmeldung: 02.03.2011
(73) Patentinhaber: Ivoclar Vivadent AG, 9494 Schaan (LI)
(72) Erfinder: Moszner, Norbert, LT-9493 Mauren (LI); Zeuner, Frank, 9488, Schellenberg (LI); Lamparth, Iris, 94723, Grabs (CH); Fischer, Urs-Karl, 9320, Arbon (CH); Rheinberger, Volker, 9490, Vaduz (LI)
(74) Vertreter: Uexküll & Stolberg

(56) Entgegenhaltungen:
- DATABASE WPI Week 200572 Thomson Scientific, London, GB; AN 2005-692783 XP002561070 & JP 2005 248137 A (MATSUKAZE KK) 15. September 2005 (2005-09-15)
- IKEMURA, ICHIZAWA, FUCHIGAMI, ITO, ENDO: "design of a new dental adhesive, effect of a water-soluble sodium acylphosphine oxide with crown ether on adhesion to dental hard tissue" DENTAL MATERIALS JOURNAL, Bd. 28, Nr. 3, 14. August 2009 (2009-08-14), Seiten 267-276, XP002561071

## Beschreibung

Die vorliegende Erfindung betrifft die Verwendung von radikalisch polymerisierbaren makrocyclischen Polyethern und polymerisierbaren makrocyclischen heteroanalogen Polyethern in Dentalmaterialien, insbesondere zur Herstellung von Adhäsiven, Beschichtungen, Zementen oder Kompositen.

Härtbare Dentalwerkstoffe auf Basis einer organischen Monomermatrix, die bei Anwendung durch radikalische Polymerisation ausgehärtet werden kann, sind bekannt. Beispiele für derartige Dentalmaterialien sind Adhäsive, Beschichtungen, Zemente oder Komposite. Bei all diesen Materialien ist ein gute Substrathaftung, sei es an Dentin- oder Zahnschmelz oder an einem anderem Dentalmaterial, eine wesentliche Eigenschaft, die aber aufgrund der schlechten Kompatibilität von organischen Monomeren bzw. Polymeren einerseits und dem Substrat, z.B. Dentin- oder Zahnschmelz, andererseits nur schwer zu erzielen ist.

Makrocyclische Polyether (Kronenether) und deren heteroanaloge Verbindungen (Coronanden oder Coranden), in denen die O-Atome teilweise oder vollständig durch Heteroatome, vor allem Stickstoff und Schwefel, ausgetauscht sind, sowie bicyclische Kronenether (Kryptanden) sind im Stand der Technik bekannt (z.B. C.J. Pedersen, H.K. Frensdorf, Angew. Chem. 84 (1972) 16). Beispiele für Kronenether oder deren heteroanaloge Verbindungen sind [15]Krone-5, [18]Krone-6 oder 1,10-Diaza-[18]krone-6:

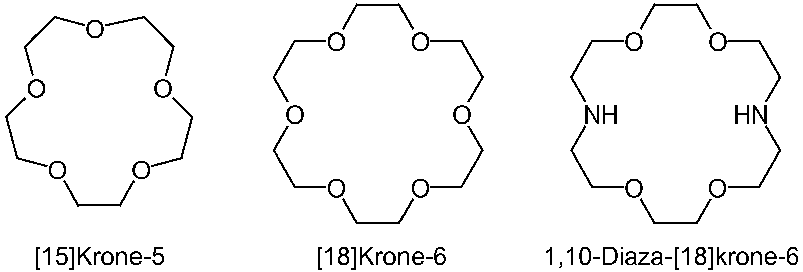

Dabei wird in einer vereinfachten Nomenklatur ein Kronenether mit x Ringgliedern und y Sauerstoffatomen als [x]Krone-y bezeichnet (vgl. Ullmann's Encyclopedia of Industrial Chemistry, 5th Ed., Band A8, 91ff). Zusätzliche Substituenten werden dem Namen als Präfix vorangestellt, wie z.B. Dibenzo[18]krone-6. Bei bicyclischen Kronenethern (Kryptanden) erfolgt die Überbrückung über zwei Stickstoffatome, wobei in der Bezeichnung die Anzahl der Sauerstoffatome der ersten, zweiten und dritten Brücke durch Punkte getrennt dem Wort Kryptand vorangestellt wird z.B. [2.2.2]Kryptand:

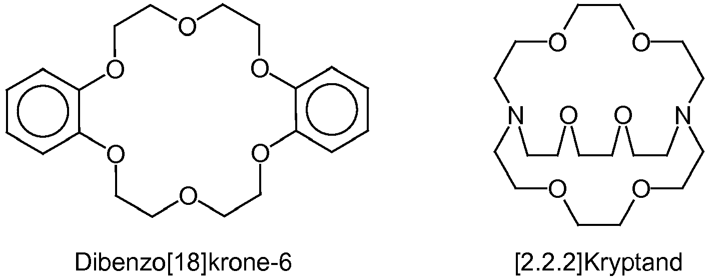

Polymerisationsfähige makrocyclische Polyether, wie z.B. polymerisationsfähige Kronenether sind ebenfalls bekannt. So werden beispielsweise ionenbindende Polymere durch radikalische Polymerisation von z.B. 4-Vinylbenzo-[15]krone-5 (VB15C5) oder 4-Vinylbenzo-[18]krone-6 (VB16C6) erhalten (S. Kopolow, T. E. Hogen Esch, J. Smid, Macromolecules 6 (1973) 133).

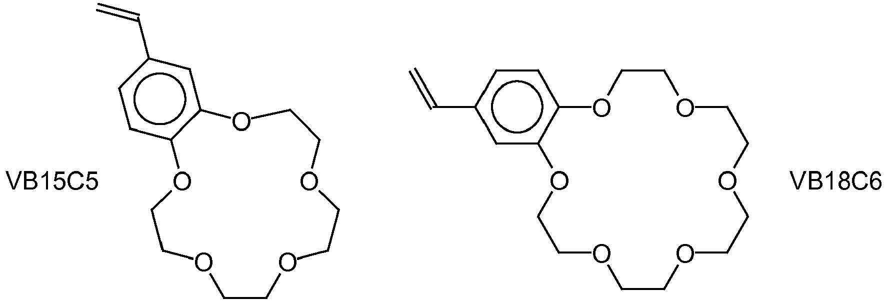

CH-A-631 344 beschreibt ein Fluoridierungsmittel für Zahnschmelz, das außer Kalium- oder Natriumfluorid und einem organischen Lösungsmittel, wie z.B. Aceton, Acetonitril oder Essigester, einen Komplexbildner, vorzugsweise einen Kronenether (z.B. [18]Krone-6) oder einen Kryptanden (z.B. [2.2.2]Kryptand) enthält. Bei den genannten makrocyclischen Polyethern handelt es sich nicht um polymerisierbare Verbindungen. Vielmehr bewirken die verwendeten Kronenether lediglich eine Auflösung des Fluorids unter Bildung von Fluoridionen ohne Solvatationshülle. Schwach solvatisierte Fluoridionen sollen vom Zahnschmelz besser aufgenommen werden.

JP 2005-248137 A beschreibt härtbare Zusammensetzungen, die eine Clathrat-Verbindung enthalten, bei der es sich insbesondere um einen Kronenether wie [18]Krone-6 handeln kann.

Ikemura et al., Dental Materials Journal, 2009, 28(3), 267-276 beschreibt Dentaladhäsive, die ein Natriumsalz eines Acylphosphinoxids wie Natrium-2,4,6-trimethyl-benzoyl-phenylphosphinoxid als radikalischen Polymerisationsinitiator in Kombination mit einem Kronenether wie [15]Krone-5 oder [18]Krone-6 enthalten.

Der vorliegenden Erfindung liegt die Aufgabe zugrunde, neuartige Dentalmaterialien mit guter Substrathaftung, guter Löslichkeit und guten mechanischen Eigenschaften bereitzustellen.

Die Aufgabe wird gelöst durch ein Dentalmaterial enthaltend mindestens 0,05 Gew.-%, bezogen auf das Gesamtgewicht des Dentalmaterials, mindestens eines radikalisch polymerisierbaren makrocyclischen Polyethers oder radikalisch polymerisierbaren makrocyclischen heteroanalogen Polyethers. Vorzugsweise enthält das erfindungsgemäße Dentalmaterial 0,05 bis 40 Gew.-%, besonders bevorzugt 1 bis 30 Gew.-% und ganz besonders bevorzugt 1 bis 20 Gew.-% radikalisch polymerisierbaren makrocyclischen Polyether und/oder makrocyclischen heteroanalogen Polyether.

Diese radikalisch polymerisierbaren makrocyclischen Polyether und makrocyclischen heteroanalogen Polyether können in der Zahntechnik und Zahnheilkunde, vor allem zur Herstellung von Adhäsiven, Beschichtungsmaterialien, Zementen und Kompositen für dentale Zwecke verwendet werden.

Der radikalisch polymerisierbare makrocyclische Polyether oder makrocyclische heteroanaloge Polyether ist eine monocyclische oder polycyclische, vorzugsweise eine monocyclische Verbindung, d.h. ein Kronenether oder heteroanaloger Kronenether, oder eine bicyclische Verbindung, d.h. ein Kryptand. Unter heteroanalogen Polyethern bzw. heteroanalogen Kronenethern versteht man im Sinne dieser Erfindung Polyether bzw. Kronenether, in denen die O-Atome teilweise oder vollständig durch andere Heteroatome, vor allem Stickstoff und/oder Schwefel, ausgetauscht sind.

In bevorzugten Ausführungsformen entspricht der radikalisch polymerisierbare makrocyclische Polyether oder makrocyclische heteroanaloge Polyether der allgemeinen Formel MC-(SP-PG)ₙ (Formel I), worin
- MC: ein n-fach substituierten Rest
(a) eines Kronenethers oder heteroanalogen Kronenethers der allgemeinen Formel IIa 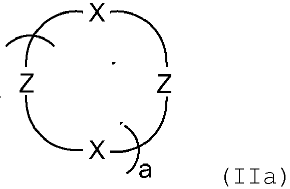 oder
(b) eines Kryptanden der Formel IIb 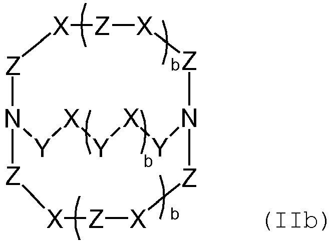 ist, worin
   X unabhängig voneinander jeweils aus O, S und NR¹ ausgewählt ist, wobei R¹ unabhängig voneinander für H, den Rest SP-PG oder einen anderen organischen Rest steht;
   Y unabhängig voneinander aus C₂-C₄-Alkylen-Resten ausgewählt ist,
   Z unabhängig voneinander jeweils aus C₁-C₄-Alkylen-, C₆-C₁₀-Arylen- und C₄-C₈-Cycloalkylen-Resten ausgewählt ist;
   a eine ganze Zahl von 3 bis 10, bevorzugt von 3 bis 6 ist und
   b jeweils unabhängig voneinander eine ganze Zahl von 1 bis 3, bevorzugt 1 oder 2 ist;
- SP: unabhängig voneinander eine Verbindungsgruppe ist oder entfällt,
- PG: eine radikalisch polymerisierbare Gruppe ist, und
- n: eine ganze Zahl von 1 bis 8, bevorzugt 1 bis 4, besonders bevorzugt 1, 2 oder 4 ist,
wobei SP-PG und/oder PG an MC über mindestens einen der Z-Reste und für X = NR¹ zusätzlich oder ausschließlich über mindestens ein N-Atom (in diesem Fall ist mindestens ein R¹ = PG oder SP-PG) gebunden ist.

Die Variable b in Formel IIb kann in allen Fällen dieselbe ganze Zahl darstellen oder für zwei oder drei unterschiedliche Zahlen stehen. Vorzugsweise hat b bei jedem Auftreten in Formel (IIb) denselben Wert. Handelt es sich bei dem radikalisch polymerisierbaren makrocyclischen Polyether oder makrocyclischen heteroanalogen Polyether um einen Kronenether bzw. heteroanalogen Kronenether, d.h. MC entspricht der Formel IIa, so gilt besonders bevorzugt a = 3, 4 oder 5. Handelt es sich bei dem radikalisch polymerisierbaren makrocyclischen Polyether um einen Kryptanden gemäß Formel IIb so ist in einer besonders bevorzugten Ausführungsform b = 2.

Die Z-Reste sind unabhängig voneinander jeweils aus C₁-C₄-Alkylen-, C₆-C₁₀-Arylen- und C₄-C₈-Cycloalkylen-Resten, vorzugsweise aus Ethylen-, Phenylen- (besonders bevorzugt 1,2-Phenylen = o-Phenylen) und Cyclohexylen-Resten (besonders bevorzugt 1,2-Cyclohexylen) ausgewählt. Die vorgenannten Reste können jeweils mit einer organischen Gruppe substituiert sein, wobei eine Substitution mit dem PG- und/oder SP-PG-Rest bevorzugt ist. Beispiel für andere geeignete Substituenten umfassen OH und COOH.

Wenn der Kronenether bzw. heteroanaloge Kronenether nur O-Atome bzw. eine Kombination von O- und S-Atomen enthält, so muss die Anbindung der n PG- und/oder SP-PG-Reste an den Makrocyclus MC über n Z-Reste erfolgen. Bevorzugt sind in diesem Fall Kronenether bzw. heteroanaloge Kronenether mit a = 4 oder 5; insbesondere Kronenether mit a = 4; Kronenether mit a = 5; S-analoge Kronenether mit a = 5, bei denen zwei O-Atome durch S-Atome ersetzt sind. Die n Z-Reste sind dann unabhängig voneinander ausgewählt aus C₁-C₄-Alkylen-, C₆-C₁₀-Arylen- und C₄-C₈-Cycloalkylen-Resten, vorzugsweise C₆-C₁₀-Arylen- und C₄-C₈-Cycloalkylen-Reste und besonders bevorzugt aus Phenylen- und Cyclohexylen-Resten. Die verbleibenden (a+1-n) Z-Reste sind vorzugsweise Ethylen.

Handelt sich um einen N-analogen Kronenether, bei dem die O-Atome teilweise oder vollständig durch NR¹-Gruppen ersetzt sind, z.B. um N-analoge Kronenether mit a = 5, bei denen die X-Gruppen eine Kombination von O-Atomen und NR¹-Gruppen sind, wie etwa solche, in denen zwei O-Atome durch NR¹-Gruppen ersetzt sind; oder N-analoge Kronenether mit a = 3, bei denen alle O-Atome durch NR¹-Gruppen ersetzt sind, so kann die Anbindung der PG- und/oder SP-PG-Reste an den Makrocyclus MC zusätzlich oder ausschließlich über die N-Atome der NR¹-Gruppe(n) erfolgen. In diesem Falle gilt für die entsprechenden NR¹-Gruppen, dass R¹ = PG und/oder SP-PG. Vorzugsweise erfolgt die Anbindung der n PG- und/oder SP-PG-Reste ausschließlich über die N-Atome, d.h. in n NR¹-Gruppen ist R¹ = PG und/oder SP-PG. Die Z-Reste sind dann typischerweise Ethylen. Es ist aber auch bei N-analogen Kronenether möglich, dass die Anbindung aller oder einiger PG- und/oder SP-PG-Reste über die Z-Reste erfolgt. Bei der alleinigen Anbindung der n PG- und/oder SP-PG-Reste über die Z-Reste sind Ausführungsformen bevorzugt, bei denen (a+1-n) Z-Reste Ethylen-Reste sind und n Z-Reste PG- und/oder SP-PG-substituierte Ethylen-Reste sind. Eine gemischte Anbindung über N-Atome und Z-Reste ist ebenso möglich.

Handelt es sich bei dem radikalisch polymerisierbaren makrocyclischen heteroanalogen Polyether um einen Kryptanden, d.h. MC entspricht der Formel IIb, so sind vorzugsweise alle X-Gruppen O-Atome.

Bei den Kryptanden erfolgt die Anbindung der n PG- und/oder SP-PG-Reste an den Makrocyclus MC vorzugsweise über n Z-Reste. Diese n Z-Reste sind dann unabhängig voneinander ausgewählt aus PG- und/oder SP-PG-substituierten C₁-C₄-Alkylen-, C₆-C₁₀-Arylen- und C₄-C₈-Cycloalkylen-Resten, vorzugsweise sind die n Z-Reste PG- und/oder SP-PG-substituiertes Phenylen und/oder Ethylen. Die verbleibenden (2b+2-n) Reste sind vorzugsweise Ethylen.

Bei Kronenethern bzw. heteroanalogen Kronenether die nur O-Atome bzw. eine Kombination von O-Atomen mit S-Atomen oder NR¹-Gruppen enthalten, ist vorzugsweise n = 1 oder 2. Bei N-analogen Kronenethern, bei denen alle O-Atome durch NR¹-Gruppen ersetzt wurden, ist vorzugsweise n = 1 oder, insbesondere für a = 3, n = 4. Bei Kryptanden ist vorzugsweise n = 1.

Bei N-analogen Kronenethern, die NR¹-Gruppen enthalten, in denen R¹ ≠ PG oder SP-PG ist, ist R¹ Wasserstoff oder ein beliebiger organischer Rest, z.B. -(CH₂)ₓ-CO-O-R² mit x = 1 bis 5, bevorzugt x = 1, und R² = H oder C₁- bis C₆-Alkyl, bevorzugt R² = H, Ethyl oder t-Butyl.

Die polymerisierbaren Gruppen PG, die an den Makrocyclus MC gebunden sind, können beliebige radikalisch polymerisierbare Gruppen sein, d.h. Gruppen, die eine ethylenisch ungesättigte Doppelbindung enthalten. Innerhalb eines Moleküls MC-(SP-PG)ₙ können für n > 1 die n PG-Reste gleich oder unterschiedlich sein. Bevorzugte polymerisierbare Gruppen sind Vinyl-, Allyl- und (Meth)acryloylgruppen, wobei die (Meth)acryloylgruppen beispielsweise Teil einer (Meth)acryloyloxy- oder (Meth)acryloylaminogruppe sein können. Diese Gruppen, insbesondere die Vinyl- und Allylgruppen, können mit weiteren organischen Resten substituiert sein, z.B. mit C₁- bis C₃-Alkyl, bevorzugt Methyl, oder -CO-O-R³, worin R³ = H oder C₁- bis C₃-Alkyl, bevorzugt R³ = H oder Ethyl ist.

Die zuvor genannten polymerisierbaren Gruppen PG können direkt oder über eine Verbindungsgruppe ("Spacer") SP an den Makrocyclus MC gebunden sein, über die Z-Reste oder N-Atome wie oben beschrieben. Innerhalb eines Moleküls MC-(SP-PG)ₙ können für n > 1 die Verbindungsgruppen gleich oder unterschiedlich sein oder sie können ganz oder teilweise entfallen, d.h. es ist innerhalb eines Moleküls möglich, das die polymerisierbaren Gruppen PG teils direkt und teils über den Spacer SP an MC gebunden sind.
Die Verbindungsgruppen SP entsprechen vorzugsweise der Formel -R⁴-Z¹-R⁵-Z²-, worin Z¹ und Z² gleich oder unterschiedlich sind und jeweils für -O-, -NH-, -CO-O-, -O-CO-, -CO-NH-, -NH-CO-, -O-CO-NH- oder -NH-CO-O- stehen oder eines oder beide von Z¹ und Z² entfallen und R⁴ und R⁵ gleich oder unterschiedlich sind und jeweils für einen C₁-C₁₀-Alkylenrest stehen oder eines oder beide von R⁴ und R⁵ entfallen. Es hat sich gezeigt, dass Moleküle MC-(SP-PG)ₙ, in denen die polymerisierbare Gruppe PG über eine Verbindungsgruppe SP an MC gebunden ist, häufig eine größere Polymerisationsfähigkeit als Moleküle aufweisen, bei denen die polymerisierbare Gruppe PG direkt an MC gebunden ist.

Beispiele für PG-Reste, die direkt oder über SP an Z-Reste gebunden sein können, umfassen: Vinyl (vorzugsweise an Z = Phenylen gebunden); Allylether (vorzugsweise an Z = Phenylen oder Ethylen gebunden); (Meth)acryloyloxyalkyl, bevorzugt (Meth)acryloyloxymethyl (vorzugsweise an Z = Phenylen, Cyclohexylen oder Ethylen gebunden), und (Meth)acryloylaminoalkyl, bevorzugt (Meth)acryloylaminomethyl und -propyl (vorzugsweise an Z = Phenylen oder Ethylen gebunden). Beispiele für PG-Reste die direkt oder über SP an N-Atome gebunden sein können, umfassen: (Meth)acryloyl, welches mit dem N-Atom des Makrocyclusses ein (Meth)acrylamid bildet; Carboxyallyl; Alkoxycarbonylallyl, bevorzugt Ethoxycarbonylallyl und t-Butoxycarbonylallyl, und (Meth)acryloylaminoalkanoyl, bevorzugt (Meth)acryloylaminohexanoyl.

Die radikalisch polymerisierbaren makrocyclischen Polyether und makrocyclischen heteroanalogen Polyether der vorliegenden Erfindung lassen sich durch bekannte Syntheseverfahren herstellen (siehe etwa C. J. Pedersen, H.K. Frensdorf, Angew. Chem. 84 (1972) 16 ff.; Studies in Organic Chemistry, Vol. 45: Crown Ethers and Analogous Compounds, Ed. M. Hiraoka, Elsevier, Amsterdam etc. 1992, 17ff.). So lassen sich z.B. polymerisationsfähige Kronenether mit einem aromatischen Ring (X = O; a = 3 bis 10; ein Z-Rest ist eine in 4-Stellung substituierte o-Phenylengruppe und die übrigen Z-Reste sind Ethylen) durch Mehrstufen-Synthese so herstellen, dass das Dichlorid Cl-(CH₂CH₂O)ₓCH₂CH₂-Cl (mit x = 2 bis 8) zunächst mit p-4-Chlormethylbrenzcatechin kondensiert wird. Der erhaltene substituierte Benzo-Kronenether wird dann mit dem Natriumsalz der Methacrylsäure in das entsprechende Methacrylat umgesetzt:

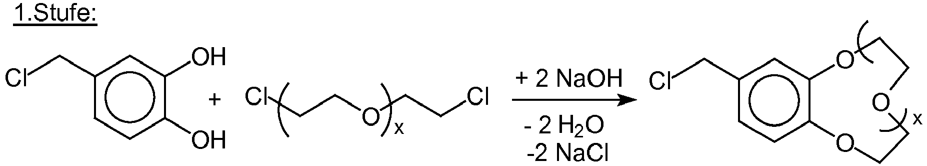

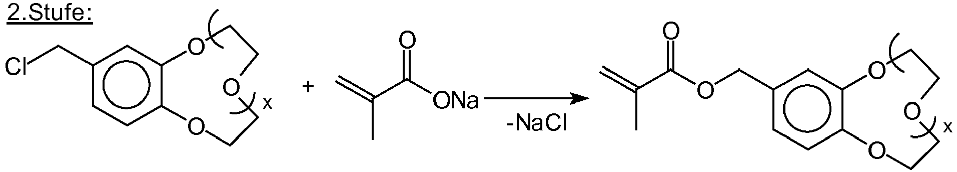

### Konkretes Beispiel: Synthese von 4-Methacryloyloxymethylbenzo-18-krone-6:

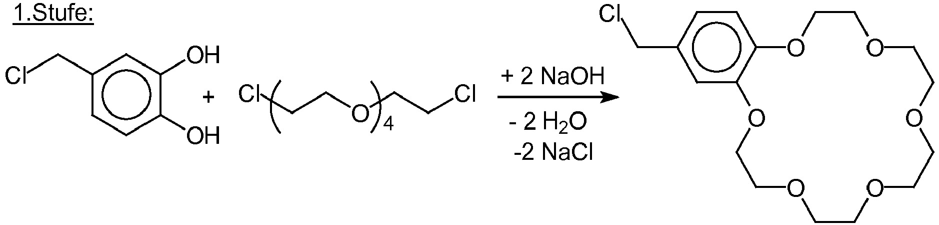

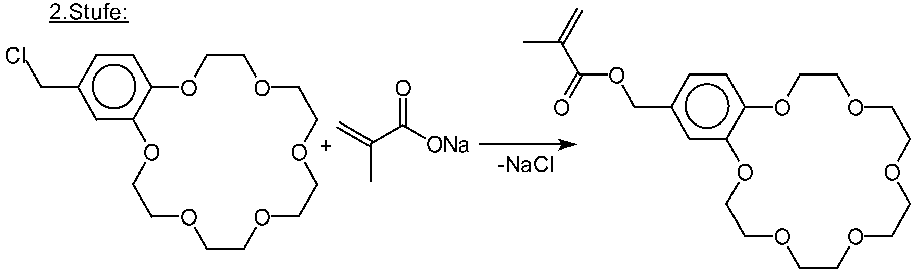

Liegen schon geeignete funktionalisierte Kronenether vor, so lassen sich diese mit aus der organischen Chemie bekannten Methoden in polymerisationsfähige Derivate umwandeln. So lassen sich beispielsweise N-analoge Kronenether, wie z.B. Tetraazamakrocyclen, einfach durch Acylierung oder Alkylierung funktionalisieren:

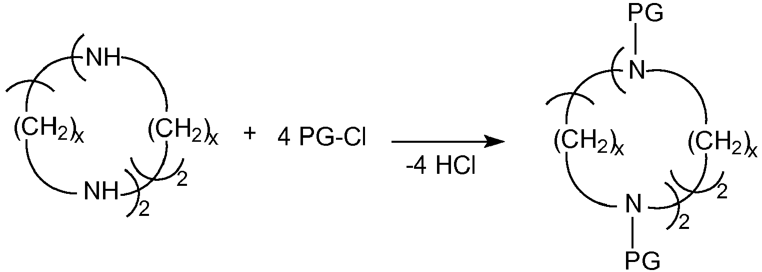

### (x = 1 bis 4, bevorzugt 2)

### Konkretes Beispiel:

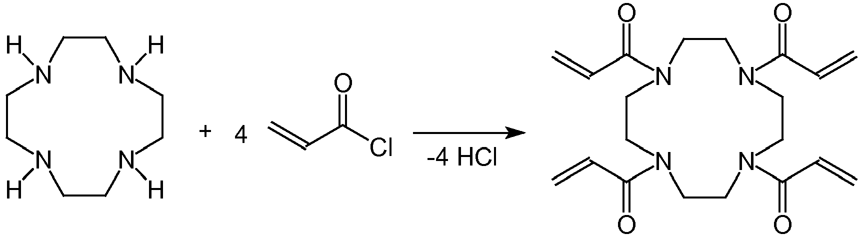

Beispiele für radikalisch polymerisierbare makrocyclische Polyether und makrocyclische heteroanaloge Polyether zur Verwendung in der vorliegenden Erfindung sind:

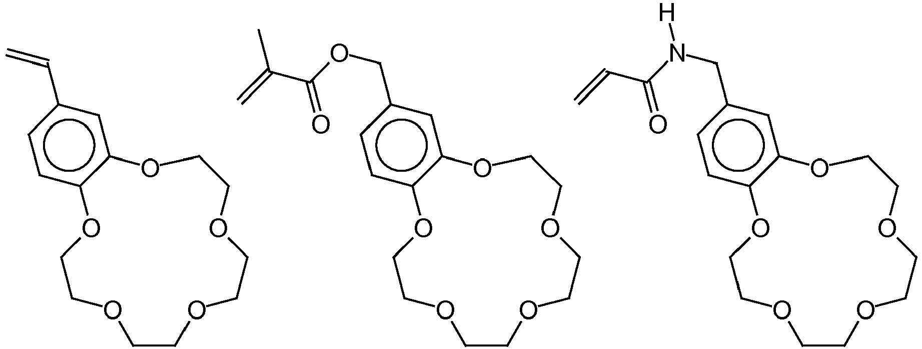

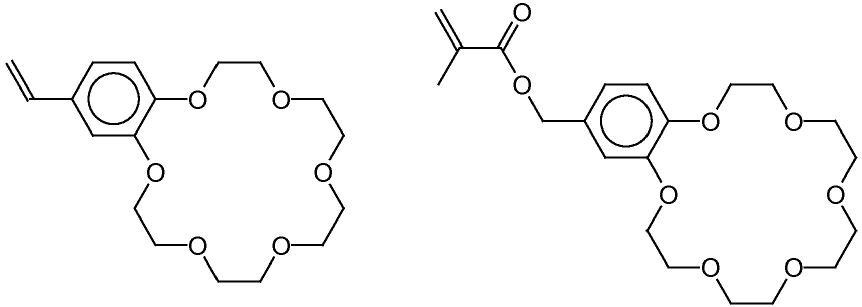

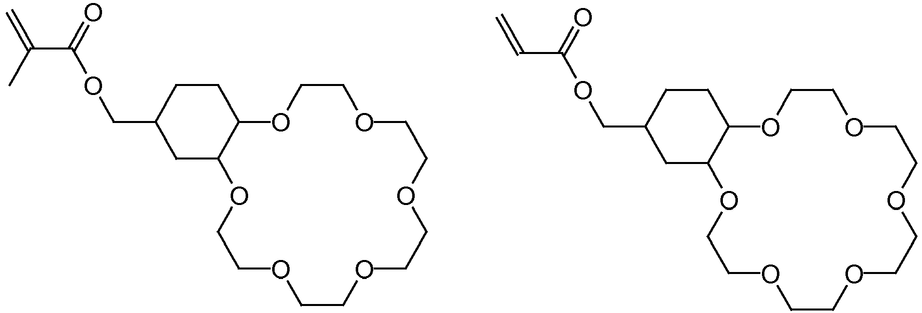

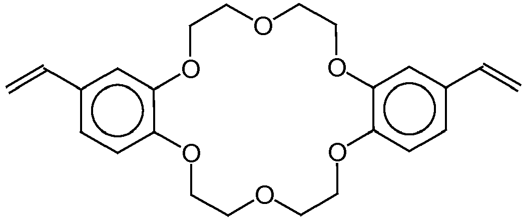

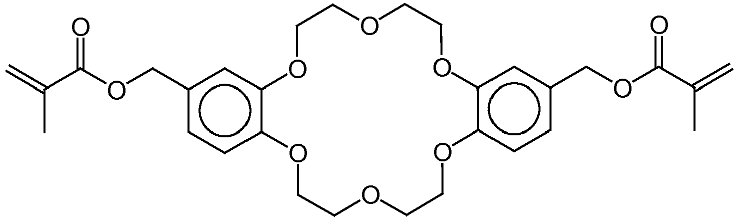

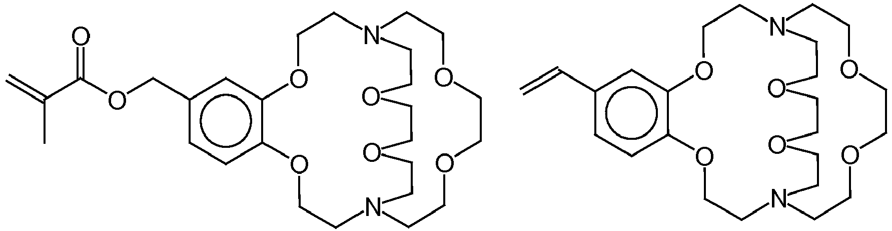

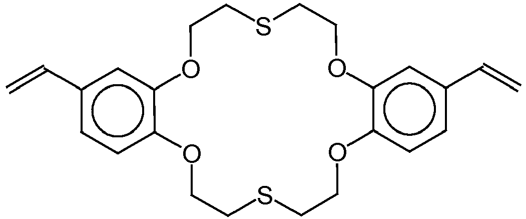

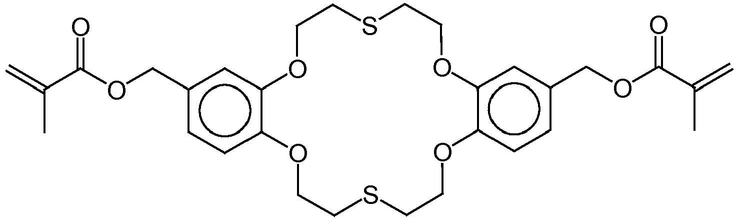

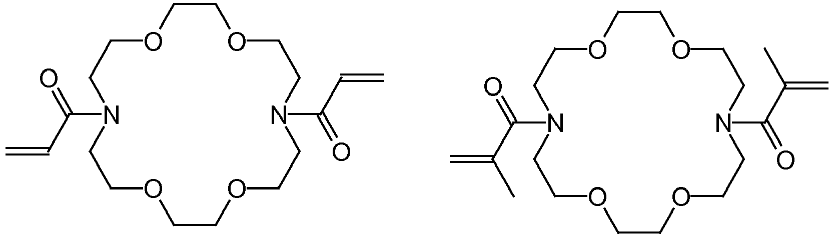

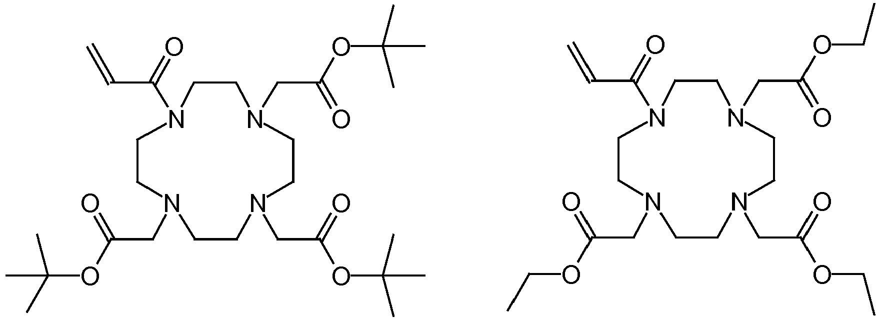

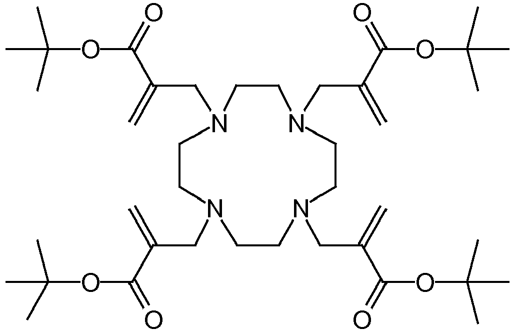

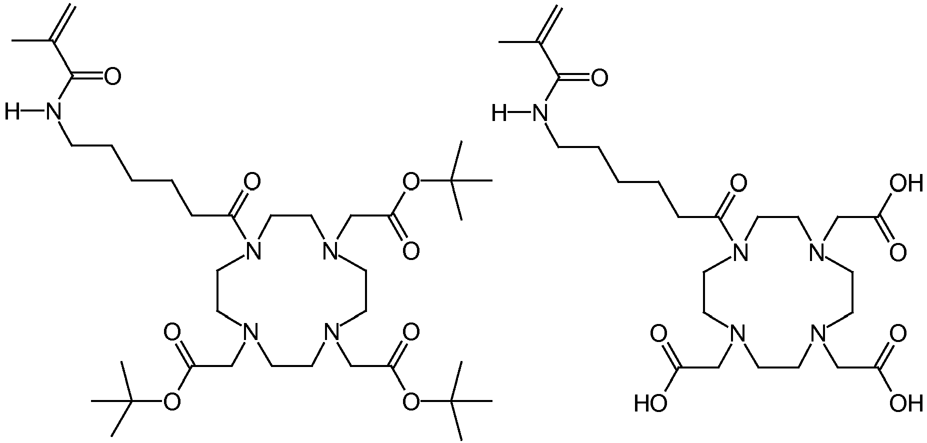

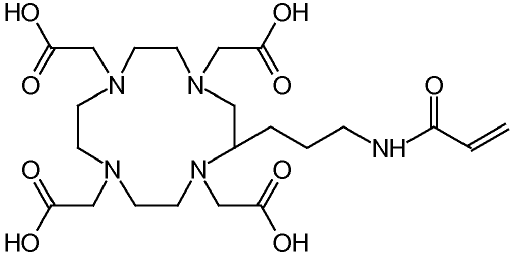

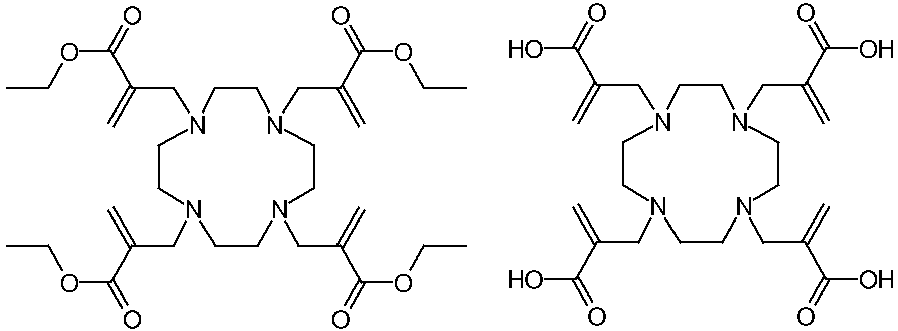

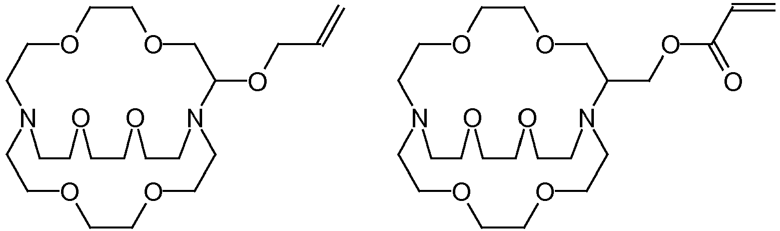

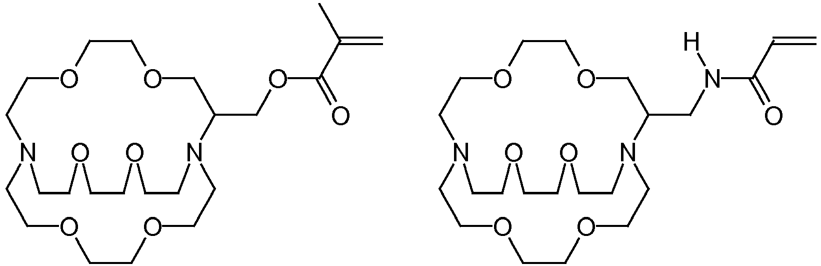

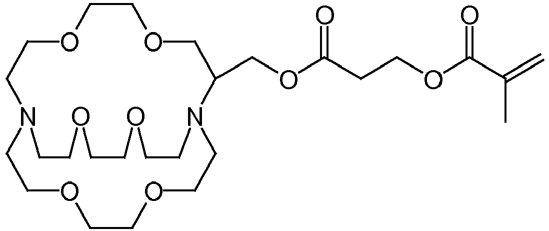

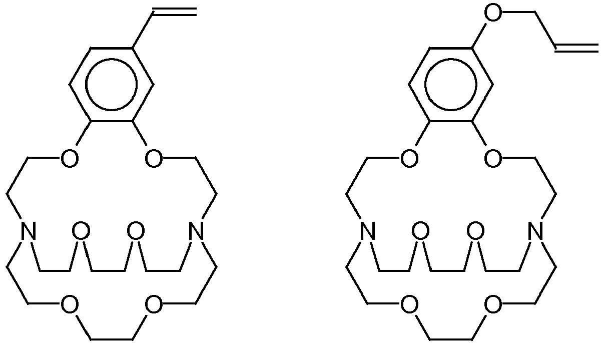

Die radikalisch polymerisierbaren makrocyclischen Polyether und/oder makrocyclischen heteroanalogen Polyether sind in den erfindungsgemäßen Dentalmaterialien typischerweise in einer Menge von 0,05 bis 40 Gew.-%, vorzugsweise 1 bis 30 Gew.-% und besonders bevorzugt 1 bis 20 Gew.-%, jeweils bezogen auf das Gesamtgewicht des Dentalmaterials, enthalten. Es kann nur eine Art von radikalisch polymerisierbarem makrocyclischem Polyether oder makrocyclischem heteroanalogem Polyether oder es können Mischungen verschiedener radikalisch polymerisierbarer makrocyclischer Polyether und/oder makrocyclischer heteroanaloger Polyether enthalten sein.

Die Dentalmaterialien gemäß der vorliegenden Erfindung sind typischerweise Adhäsive, Beschichtungsmaterialien, Zemente oder Komposite für dentale Zwecke.

Die radikalisch polymerisierbaren makrocyclischen Polyether oder makrocyclischen heteroanalogen Polyether in den erfindungsgemäßen Dentalmaterialien sind sehr gut in Wasser oder Mischungen von Wasser mit polaren Lösungsmitteln, wie Aceton, Ethanol oder Acetonitril, löslich. Als Phasentransfer-Katalysatoren fördern sie die Benetzung von organischen Komponenten auf unterschiedlichsten Substratoberflächen bzw. die Diffusion von Verbindungen in das Innere poröser Materialien. Darüber hinaus können die radikalisch polymerisierbaren makrocyclischen Polyether und makrocyclischen heteroanalogen Polyether Metallionen, wie z.B. Calciumionen, komplexieren und sind deshalb in der Lage, die Haftung auf Zahnschmelz und Dentin zu vermitteln. In den erfindungsgemäß verwendeten Monomeren sind die Makrocyclen kovalent direkt oder über Verbindungsgruppen mit den polymerisationsfähigen Gruppen verbunden, so dass die makrocyclischen Bausteine nach Polymerisation in das Polymerisat kovalent eingebunden sind, was die Biokompatibilität der entsprechenden Materialien verbessert.

Die erfindungsgemäßen Dentalmaterialien können weiterhin zusätzliche radikalisch polymerisierbare Matrixmonomere enthalten, die sich von dem radikalisch polymerisierbaren makrocyclischen Polyether oder makrocyclischen heteroanalogen Polyether unterscheiden. Es versteht sich, dass Mischungen von verschiedenen zusätzlichen Monomeren oder auch nur eine Art eines zusätzlichen Monomers vorhanden sein können.

Beispiele für geeignete zusätzliche radikalisch polymerisierbare Monomere sind mono- oder polyfunktionelle (Meth)acrylate, wie etwa Methyl-, Ethyl-, Hydroxyethyl-, Butyl-, Benzyl-, Tetrahydrofurfuryl- und Isobornyl(meth)acrylat, Bisphenol-A-di(meth)acrylat, Bis-GMA (ein Additionsprodukt aus Methacrylsäure und Bisphenol-A-diglycidylether), UDMA (ein Additionsprodukt aus 2-Hydroxyethylmethacrylat und 2,2,4-Trimethylhexamethylendiisocyanat), Di-, Tri- oder Tetraethylenglykoldi(meth)acrylat, Trimethylolpropantri(meth)acrylat, Pentaerythrittetra(meth)acrylat, sowie Glycerindimethacrylat, 1,4-Butandioldi(meth)acrylat, 1,10-Decandioldi(meth)acrylat und 1,12-Dodecandioldi(meth)acrylat.

Geeignete zusätzliche radikalisch polymerisierbare Monomere sind auch hydrolysestabile Verdünnermonomere, wie etwa hydrolysestabile Mono(meth)acrylate, z.B. Mesitylmethacrylat oder 2-(Alkoxymethyl)acrylsäuren, z.B. 2-(Ethoxymethyl)acrylsäure, 2-(Hydroxymethyl)acrylsäure, N-mono- und -disubstitiuierte Acrylamide, wie z.B. N-Ethylacrylamid, N,N-Dimethacrylamid, N-(2-Hydroxyethyl)acrylamid und N-Methyl-N-(2-hydroxyethyl)-acrylamid, bzw. N-monosubstituierte Methacrylamide, wie z.B. N-Ethylmethacrylamid und N-(2-Hydroxyethyl)methacrylamid sowie außerdem N-Vinylpyrrolidon und Allylether.

Beispiele für hydrolysestabile Vernetzermonomere, die ebenfalls in den erfindungsgemäßen Dentalmaterialien eingesetzt werden können, sind Urethane aus 2-(Hydroxymethyl)acrylsäureestern und Diisocyanten, wie 2,2,4-Trimethylhexamethylendiisocyanat oder Isophorondiisocyanat, vernetzende Pyrrolidone, wie z.B. 1,6-Bis(3-vinyl-2-pyrrolidonyl)-hexan, und kommerziell zugängliche Bisacrylamide wie Methylen- und Ethylenbisacrylamid, bzw. Bis(meth)acrylamide, wie z.B. N,N'-Diethyl-1,3-bis(acrylamido)-propan, 1,3-Bis(methacrylamido)-propan, 1,4-Bis(acrylamido)-butan und 1,4-Bis(acryloyl)-piperazin, die durch Umsetzung aus den entsprechenden Diaminen mit (Meth)acrylsäurechlorid synthetisiert werden können.

Weiterhin lassen sich als zusätzliche radikalisch polymerisierbare auch bekannte schrumpfungsarme radikalisch ringöffnend polymerisierbare Monomere wie z.B. mono- oder multifunktionelle Vinylcyclopropane bzw. bicyclische Cyclopropanderivate (siehe DE-C-196 16 183 bzw. EP 1 413 569 A) oder cyclische Allylsulfide (siehe US 6,043,361 oder US 6,344,556) einsetzen, die darüber hinaus auch in Kombination mit den voranstehend aufgeführten Di(meth)acrylat-Vernetzern verwendet werden können. Geeignete ringöffnend polymerisierbare Monomere sind insbesondere Vinylcyclopropane, wie 1,1-Di(ethoxycarbonyl)- und 1,1-Di(methoxycarbonyl)-2-vinylcyclopropan, und die Ester der 1-Ethoxycarbonyl- und 1-Methoxycarbonyl-2-vinylcyclopropancarbonsäure mit Ethylenglycol, 1,1,1-Trimethylolpropan, 1,4-Cyclohexandiol oder Resorcin. Geeignete bicyclische Cyclopropanderivate sind 2-(Bicyclo[3.1.0]hex-1-yl)acrylsäuremethyl- und -ethylester und deren Disubstitutionsprodukte in 3-Stellung wie (3,3-Bis(ethoxycarbonyl)bicyclo[3.1.0]hex-1-yl)acrylsäuremethyl- oder -ethylester. Geeignete cyclische Allylsulfide sind die Additionprodukte von 2-(Hydroxymethyl)-6-methylen-1,4-dithiepan oder 7-Hydroxy-3-methylen-1,5-dithiacylooctan mit 2,2,4-Trimethylhexamethylen-1,6-diisocyanat oder dem asymmetrischen Hexamethylendiisocyanat-Trimeren (das asymmetrische Trimer zeigt eine Iminooxadiazindion-Struktur im Gegensatz zum symmetrische Hexamethylendiisocyanat-Trimer mit Isocyanurat-Struktur). Asymmetrische Hexamethylendiisocyanat-Trimere sind beispielsweise unter der Bezeichnung Desmodur® VP LS 2294 von der Bayer AG kommerziell erhältlich.

Außerdem können als zusätzliche Monomere radikalisch polymerisierbare Polysiloxane eingesetzt werden, die aus geeigneten Methacrylsilanen, wie z.B. 3-(Methacryloyloxy)propyltrimethoxysilan, hergestellt werden können und z.B. in DE-C-199 03 177 beschrieben sind.

Schließlich lassen sich als zusätzliche radikalische polymerisierbare Matrixmonomere auch Mischungen der voranstehend genannten Monomeren mit radikalisch polymerisierbaren, säuregruppenhaltigen Haftmonomeren verwenden. Geeignete säuregruppenhaltige Monomere sind polymerisationsfähige Carbonsäuren, wie Maleinsäure, Acrylsäure, Methacrylsäure, 2-(Hydroxymethyl)acrylsäure, 4-(Meth)acryloyloxyethyltrimellitsäureanhydrid, 10-Methacryloyloxydecylmalonsäure, N-(2-Hydroxy-3-methacryloyloxypropyl)-N-phenylglycin bzw. 4-Vinylbenzoesäure. Beispiele für geeignete Phosphonsäuremonomere sind Vinylphosphonsäure, 4-Vinylphenylphosphonsäure, 4-Vinylbenzylphosphonsäure, 2-Methacryloyloxyethylphosphonsäure, 2-Methacrylamidoethylphosphonsäure, 4-Methacrylamido-4-methylpentyl-phosphonsäure, 2-[4-(Dihydroxyphosphoryl)-2-oxa-butyl]-acrylsäure und 2-[2-Dihydroxyphosphoryl)-ethoxymethyl]-acrylsäure-ethyl- und -2,4,6-trimethylphenylester. Beispiele für geeignete acide polymerisationsfähige Phosphorsäureester sind 2-Methacryloyloxypropylmono- und -dihydrogenphosphat, 2-Methacryloyloxyethylmono- und -dihydrogenphosphat, 2-Methacryloyloxyethylphenyl-hydrogenphosphat, Dipentaerythritol-pentamethacryoyloxyphosphat, 10-Methacryloyloxydecyl-dihydrogenphosphat, Dipentaerythritolpentamethacryloyloxyphosphat, Phosphorsäuremono-(1-acryloyl-piperidin-4-yl)-ester, 6-(Methacrylamido)hexyldihydrogenphosphat und 1,3-Bis-(N-acryloyl-N-propyl-amino)-propan-2-yl-dihydrogenphosphat. Beispiele für geeignete polymerisationsfähige Sulfonsäuren sind Vinylsulfonsäure, 4-Vinylphenylsulfonsäure und 3-(Methacrylamido)-propylsulfonsäure.

Typischerweise enthalten die erfindungsgemäßen Dentalmaterialien einen Initiator für die radikalische Polymerisation. Der Initiator wird ausgewählt, je nachdem, welche Art von Härtung ob für den erfindungsgemäßen Dentalwerkstoff erwünscht ist, z.B. Strahlungshärtung (Photopolymerisation) und/oder Heißhärtung und/oder Härtung bei Raumtemperatur.

Zur Initiierung der radikalischen Photopolymerisation werden beispielsweise Benzophenon, Benzoin sowie deren Derivate oder α-Diketone oder deren Derivate wie 9,10-Phenanthrenchinon, 1-Phenyl-propan-1,2-dion, Diacetyl oder 4,4-Dichlorbenzil eingesetzt. Bevorzugt werden Campherchinon oder 2,2-Methoxy-2-phenyl-acetophenon und besonders bevorzugt α-Diketone in Kombination mit Aminen als Reduktionsmittel, wie z.B. 4-(Dimethylamino)-benzoesäureester wie p-N,N-Dimethylaminobenzoesäureethylester, N,N-Dimethylaminoethylmethacrylat, N,N-Dimethyl-sym.-xylidin oder Triethanolamin, verwendet. Besonders geeignet sind auch Norrish-Typ-I-Photoinitiatoren vor allem Acyl- der Bisacylphosphinoxide, Monoacyltrialkyl- bzw. Diacyldialkylgermanium-Verbindungen, wie z.B. Benzoyltrimethylgermanium, Dibenzoyldiethylgermanium und Bis(4-methoxybenzoyl)diethylgermanium. Dabei lassen sich auch Mischungen der verschiedenen Photoinitiatoren einsetzen, wie z.B. Dibenzoyldiethylgermanium in Kombination mit Campherchinon und 4-Dimethylaminobenzoesäureethylester.

Als Initiatoren für die Heißhärtung eignen sich z.B. Benzpinakol oder 2,2'-Dialkylbenzpinakole.

Als Initiatoren für eine bei Raumtemperatur durchgeführte Polymerisation werden typischerweise Redox-Initiatorkombinationen, wie z.B. Kombinationen von Benzoylperoxid mit N,N-Dimethyl-sym.-xylidin oder N,N-Dimethyl-p-toluidin, verwendet. Darüber hinaus sind auch Redoxsysteme bestehend aus Peroxiden und solchen Reduktionsmitteln, wie z.B. Ascorbinsäure, Barbiturate oder Sulfinsäuren, besonders geeignet.

Die Gesamtinitiatormenge beträgt typischerweise 0,01 bis 10 Gew.-%, bevorzugt 0,1 bis 3,0 Gew.-% und besonders bevorzugt, 0,1 bis 1,0 Gew.-%, jeweils bezogen auf das Gesamtgewicht des Dentalmaterials.

Weiterhin können die erfindungsgemäßen Dentalmaterialien optional zur Verbesserung der mechanischen Eigenschaften oder zur Einstellung der Viskosität mit organischen oder anorganischen Partikeln gefüllt sein. Die Füllstoffe haben vorzugsweise eine Partikelgröße von 5 nm bis 2.000 µm, vorzugsweise 10 nm bis 1.000 µm. Bevorzugte anorganische partikuläre Füllstoffe sind amorphe kugelförmige Materialien auf der Basis von Oxiden, wie ZrO₂ und TiO₂ bzw. Mischoxiden aus SiO₂, ZrO₂ und/oder TiO₂, nanopartikuläre oder mikrofeine Füllstoffe, wie pyrogene Kieselsäure und Fällungskieselsäure, sowie Minifüllstoffe, wie Quarz-, Glaskeramik- und Glaspulver mit einer durchschnittlichen Teilchengröße von 0,01 bis 1 µm sowie röntgenopake Füllstoffe, wie Ytterbiumtrifluorid und nanopartikuläres Tantal(V)-oxid bzw. Bariumsulfat. Es können als Füllstoff auch Mischungen verschiedener Füllstoffe eingesetzt werden. Im Sinne dieser Erfindung versteht man unter nanopartikulären Füllstoffen Füllstoffe mit einer Partikelgröße von 5 bis 10 nm, unter mikrofeinen Füllstoffen Füllstoffe mit einer Partikelgröße von 10 nm bis 100 nm und unter Minifüllstoffen Füllstoffe mit einer Partikelgröße von 10 bis 1.000 nm. Gegebenenfalls - vorzugsweise, wenn es sich bei dem Dentalmaterial um ein Adhäsiv oder ein Beschichtungsmaterial handelt - kann das erfindungsgemäße Dentalmaterial Lösungsmittel oder eine Mischung von Lösungsmittel enthalten. Bevorzugt sind Wasser, Ethanol und Mischungen von Wasser mit Aceton und/oder Ethanol.

Optional können die erfindungsgemäßen Dentalmaterial ein oder mehrere weitere Additive enthalten, z.B. Stabilisatoren, Aromastoffe, Farbmittel, mikrobiozide Wirkstoffe, fluoridionenabgebende Additive, optische Aufheller, Weichmacher und UV-Absorber.

Die radikalisch polymerisierbaren makrocyclischen Polyether oder makrocyclischen heteroanalogen Polyether können beispielsweise in reiner Form oder im Falle von Kompositen oder Zementen auch als Lösung in Monomeren, im Falle von Adhäsiven oder Beschichtungsmaterialien als Lösung in einem geeigneten Lösungsmittel in die dentale Zusammensetzung eingearbeitet werden. Das Einarbeiten erfolgt vorzugsweise mittels üblicher Dispergiermethoden, wie z.B. Rühren oder Kneten.

In typischen Ausführungsformen enthält das Dentalmaterial gemäß der vorliegenden Erfindung:
(i) 0,05 bis 40 Gew.-%, bevorzugt 1 bis 30 Gew.-% und besonders bevorzugt 1 bis 20 Gew.-% des radikalisch polymerisierbaren makrocyclischen Polyethers und/oder makrocyclischen heteroanalogen Polyethers,
(ii) 0,01 bis 10 Gew.-%, besonders bevorzugt 0,1 bis 3,0 Gew.-% eines Initiators für die radikalische Polymerisation,
(iii) 0 bis 80 Gew.-%, bevorzugt 0 bis 60 Gew.-% und besonders bevorzugt 5 bis 50 Gew.-% mindestens eines radikalisch polymerisierbaren Monomers, das sich von Komponente (i) unterscheidet,
(iv) 0 bis 75 Gew.-%, in Abhängigkeit von der Anwendung bevorzugt 0 bis 20 Gew.-% (bei Adhäsiven und Beschichtungsmaterialen) oder 20 bis 75 Gew.-% (bei Zementen und Kompositen) Füllstoff,
(v) 0 bis 95 Gew.-%, bevorzugt 0 bis 70 Gew.-% und besonders bevorzugt 5 bis 50 Gew.-% Lösungsmittel bei Adhäsiven oder Beschichtungsmaterialien,
jeweils bezogen auf das Gesamtgewicht des Dentalmaterials.

Die Erfindung wird im Folgenden anhand von Beispielen näher erläutert.

### Beispiel 1:

### Synthese von 4-(Methacryloyloxymethyl)-benzo-15-krone-5 (MA15-K5)

Die Synthese des polymerisationsfähigen Kronenethers MA15-K5 erfolgte in 2.Stufen anlog der Literatur (A. J. Varma, T. Majewicz, J. Smid, J. Polym. Sci., Polym. Chem. Ed., 17 (1979) 1573):

### 1.Stufe: 4-Hydroxymethyl-benzo-15-krone-5

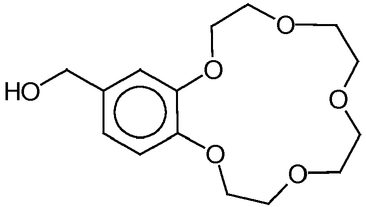

Zu einer Suspension von 4-Formyl-benzo-15-krone-5 (16,24 g, 54,8 mmol), das entsprechend A. J. Varma, J. Smid, J. Polym. Sci., A-1, 15 (1977) 1189 hergestellt wurde, in 300 ml absolutem Ethanol wurde unter Rühren Natriumborhydrid (2,49 g, 65., mmol) gegeben und 24 h nachreagieren gelassen. Es wurde mit 300 ml Wasser gequencht und mit 15 ml 25 %iger Essigsäure neutralisiert. Die Wasserphase wurde 4 mal mit jeweils 125 ml Chloroform extrahiert und die vereinigten organischen Phasen über wasserfreiem Na₂SO₄ getrocknet und filtriert. Das Filtrat wurde am Rotationsverdampfer eingeengt und am Feinvakuum getrocknet. Man erhielt 15,60 g eines gelblichen Öls, dass in 10 ml Aceton gelöst und über eine mit Kieselgel gefüllte Fritte filtriert (Kieselgel 60, 0,035-0,070 mm, 60 g, Ø 50 mm; THF, Elutionsvolumen: 600 ml) wurde. Nach erneutem Einengen und Trocknen im Feinvakuum wurden 14,88 g (49,9 mmol, 91 % Ausbeute) eines farblosen Öl (erstarrt langsam zu weißem Feststoff, HPLC-Reinheit: 95,7 %) erhalten.
¹H-NMR (CDCl₃, 400 MHz): δ = 2,75 (b, 1 H, OH), 3,75 (s, 8 H, CH₂O(C₂H₄O)₂CH₂), 3,87 (q, 4 H, ArOCH₂CH₂), 4,09 (q, 4 H, ArOCH₂CH₂), 4,55 (s, 2 H, CH₂Ar), 6, 79-6, 86 (m, 3 H, ^{2,5,6}H-Ar).
IR (KBr): 3431, 2930, 2875, 1592, 1515, 1451, 14261370, 13561325, 1287, 1259, 1236, 1161, 1128, 1088, 1045, 960, 935, 880, 844, 825, 804, 784, 745 cm⁻¹.

### 2.Stufe: 4-(Methacryloyloxymethyl)-benzo-15-krone-5 (MA15-K5)

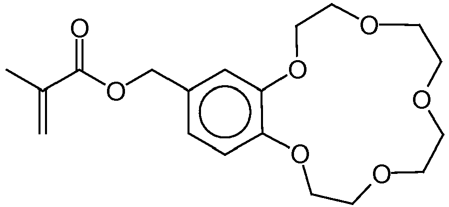

Eine Lösung von 4-(Hydroxymethyl)-benzo-15-krone-5 (7,68 g, 25,7 mmol), Triethylamin (2,61 g (25,7 mmol) und 4-Dimethylamino-pyridin (122 mg, 1,0 mmol) in 150 ml Dichlormethan) wurde auf -5 °C abgekühlt und dann unter Rühren eine Lösung von Methacrylsäureanhydrid (3,97 g, 25,7 mmol) und BHT (Butylhydroxytoluol) (10 mg) in 30 ml Dichlormethan innerhalb 10 min zugetropft. Die klare farblose Lösung wurde noch 1 h bei -5 °C und 18 h bei Raumtemperatur gerührt und danach 3 mal mit je 100 ml Wasser gewaschen. Die vereinigten Wasserphasen wurden 2 mal mit je 50 ml Dichlormethan extrahiert und die vereinigten organischen Phasen über wasserfreiem Na₂SO₄ getrocknet. Es wurde filtriert, nach Zugabe von 5 mg BHT am Rotationsverdampfer eingeengt und am Feinvakuum getrocknet. Man erhielt 8,95 g eines leicht rötlichen Feststoffs der in 10 ml Dichlormethan gelöst und mittels MPLC gereinigt (Kieselgel 60, 0,015-0,040 mm, 85 g, Ø 40 mm x 15 cm; Eluent: n-Hexan/Ethylacetat 1:1) wurde. Es wurden 5,68 g (15,5 mmol; 60 % Ausbeute) eines weißen Feststoffs (HPLC-Reinheit: 97,4 %) erhalten. Schmelzpunkt: 75,2 °C-76,6 °C.
¹H-NMR (CDCl₃, 400 MHz): δ = 1,95 (s, 3 H, CH₃), 3,77 (s, 8 H, C₂H₄OC₂H₄OC₂H₄), 3,91 (q, 4 H, CH₂CH₂OAr), 4,14 (q, 4 H, CH₂OAr), 5,10 (s, 2 H, CH₂OC=O), 5,57 und 6,12 (s, 2 x 1 H, =CH₂), 6,83 - 6, 94 (m, 3 H, ^{2,5,6}H-Ar).
IR (KBr): 2927, 2871, 1712, 1635, 1592, 1524, 1457, 1431, 1343, 13161268, 1246, 1165, 1133,1116, 1081, 1054, 1007, 979, 953, 932, 896, 876, 815, 798, 786, 742, 639 cm⁻¹.

### Beispiel 2:

### Synthese von 2-[4,7,10-Tris-(2-tert-butoxycarbonyl-allyl)-1,4,7,10-tetraazacyclododec-1-ylmethyl]-acrylsäure-tert-butyl-ester (V-621)

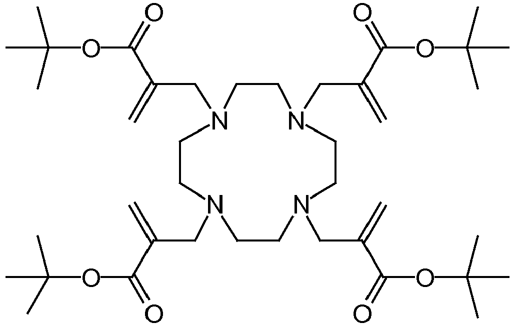

Cyclen (1,4,7,10-Tetraazacyclododecan) (8,6 g, 0,05 mol) wurde in 250 ml wasserfreiem Acetonitril gelöst, mit Kaliumcarbonat (27,6 g, 0,2 mol) versetzt und dann unter Rühren und Eisbadkühlung eine Lösung von 2-Brommethylacrylsäure-tert-butylester (44,2 g, 0,2 mol) und 20 mg BHT in 125 ml wasserfreiem Acetonitril zugetropft. Es wurde über Nacht bei Raumtemperatur nachreagieren gelassen und dann der ausgefallene Niederschlag abgesaugt, in 250 ml deionisiertem Wasser suspendiert und 1 h kräftig verrührt. Es wurde erneut abgesaugt, mit etwas Wasser gewaschen und im Vakuumtrockenschrank bei 50 °C getrocknet. Man erhielt 29,0 g (79 % Ausbeute) eines weißen Feststoffes (Schmp.: 121-122 °C).
¹H-NMR (CDCl₃, 400 MHz): δ = 1,49 (s, 36 H, CH₃), 2,58 (s, 16 H CH₂CH₂), 3,10 (s, 8 H, CH₂C=), 5,97 und 6,12 (s, 2 x 4 H, CH₂=).
IR (KBr): 2976, 2950, 2810, 1701, 1636, 1477, 1453, 1434, 1389, 1368, 1354, 1318, 1295, 1257, 1129, 1078, 1017, 992, 975, 954, 940, 922, 852, 823, 761, 679, 648 cm⁻¹.

### Beispiel 3:

### Synthese von 1-Acryloyl-,4,7,10-tris(tert-butoxycarbonyl-methyl)-1,4,7,10-tetraazacyclododecan (MA-439)

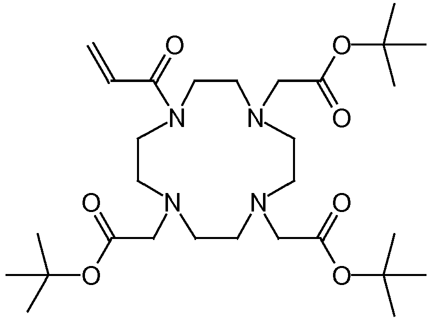

Zu einer Suspension von 1,4,7-Tris(tert-butoxycarbonylmethyl)-1,4,7,10-tetraazacyclododecan-hydrobromid (8,45 g, (14,2 mmol), Natriumhydroxid (1,25 g 31,2 mmol) und 60 ml deionisiertem Wasser wurde bei -5 bis 0 °C innerhalb 90 min eine Lösung von frisch destilliertem Acrylsäurechlorid (1,41 g, 15,6 mm) in 10 mg BHT und 200 ml Dichlormethan (getrocknet über Molsieb 4 Å) getropft. Nach beendeter Zugabe wurde das Reaktionsgemisch 1 h bei 0 °C gerührt, dann das Eisbad entfernt und 3 h bei Raumtemperatur weitergerührt. Die wässrige Phase wurde durch Zugabe von 10 g NaCl gesättigt und 2 mal mit jeweils 40 ml Dichlormethan extrahiert. Die vereinigten organischen Phasen wurden über wasserfreiem Na₂SO₄ getrocknet, filtriert und nach Zugabe von 5 mg BHT am Rotationsverdampfer eingeengt und im Feinvakuum nachgetrocknet. Man erhielt 8,23 g eines hochviskosen Rohprodukts, das säulenchromatographisch (Kieselgel 60, 0,035-0,070 mm) erst mit Aceton und dann mit n-Hexan/Ethylacetat 1:1 als Eluent aufgereinigt wurde. Nach dem Einengen unter Zusatz von BHT und Trocknen bei 50 °C wurden 4,77 g (8,4 mmol, 59 % Ausbeute) eines hochviskosen farblosen Öls erhalten (HPLC-Reinheit: 96 %).
¹H-NMR (CDCl₃, 400 MHz): δ = 1,44, 1, 45 und 1,46 (s, 3 x 9 H, CH₃), 2,70-2,77 (m, 8 H, O=CCH₂NC₂H₄NCH₂C=O), 2,96 und 3,02 (m 2x 2H, CH₂CH₂NC=O), 3,27, 3, 30 und 3,32 (s, 3 x 2 H, alle CH₂C=O), 3,61 und 3,75 (t, 2 x 2 H, CH₂NC=O), 5,60-5,65, 6,28-6,35 und 6,56-6,65 (m, 3 x 1 H, CH=CH₂).
IR (neat): 2976, 2931, 2824, 1722, 1647, 1610, 14401392, 1366, 1290, 1248, 1216, 1146, 1051, 979, 950, 914, 847, 795, 744.

### Beispiel 4:

### Synthese von {4,7-Bis-tert-butoxycarbonylmethyl-10-[6-(2-methyl-acryloylamino)-hexanoyl]-1,4,7,10-tetraazacyclododec-1-yl}-essigsäure-tert.-butylester (MA-442)

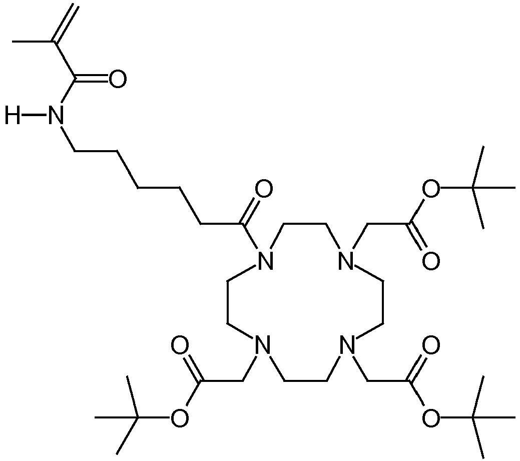

Eine Suspension von 6-(Methacryloylamino)-capronsäure (2,80 g, 14,0 mmol), 1,4,7-Tris(tert-butoxycarbonylmethyl)-1,4,7,10-tetraazacyclododecan-hydrobromid (7,75 g, 13,0 mmol), N,N-Dimethylaminopyridin (1,83 g, 15,0 mmol) und 5 mg BHT in 60 ml Dichlormethan wurde auf - 5°C abgekühlt und unter Rühren portionsweise mit 1-Ethyl-3-(3-dimethylaminopropyl)carbodiimid-hydrochlorid (2,88 g, 15,0 mmol) versetzt. Zur vollständigen Umsetzung wurde 2 h bei -5°C und bei Raumtemperatur über Nacht gerührt. Dann wurde noch 3 mal im Abstand von 2 h je 1-Ethyl-3-(3-dimethyl-aminopropyl)carbodiimid-hydrochlorid (1,00 g, 6,44 mmol) und N,N-Dimethylaminopyridin (0,6 g, 4,9 mmol) versetzt und abschliessend 6 h rühren lassen. Die Lösung wurde mit 3 mal jeweils 50 ml gesättigter wässriger NaCl-Lösung gewaschen, über wasserfreiem Na₂SO₄ getrocknet, filtriert, nach Zugabe von 5 mg BHT am Rotationsverdampfer eingeengt und am Feinvakuum getrocknet. Man erhielt 10,3 g eines gelblichen Öls, dass chromatographisch 2 mal gereinigt wurde (Kieselgel 60, 0,015-0,040 mm, Aceton). Das Aceton wurde nach Zugabe von 5 mg BHT am Rotationsverdampfer wieder abdestilliert und das Produkt im Feinvakuum getrocknet. Man erhielt 6,13 g (8,8 mmol, 68 % Ausbeute) eines gelben hochviskosen Öls (HPLC-Reinheit: 95 %).
¹H-NMR (CDCl₃, 400 MHz): δ = 1,31-1,72 (m, 6 H, (CH₂)₃CH₂N, 1,46 (s, 27 H, t-Bu), 1,96 (s, 3 H, CH₃C=), 2,32 (t, 2 H, CH₂CH₂C=O) 2,73, (b, 6 H, CH₂N), 2,81, 2,89 und 2,98 (b, 3 x 2 H, CH₂N), 3,27 und 3,32 (b, 2 x 4 H, CH₂N), 3,54 und 3,66 (b, 2 x 2H, CH₂N), 5,30 und 5,69 (s, 2 x 1 H, CH₂=), 6,19 (b, 1 H, NH).
IR: 3320, 2970, 2931, 2860, 1724, 1619, 1530, 1455, 1392, 1366, 1305, 1216, 1147, 934, 920, 846, 744 cm⁻¹.

### Beispiel 5:

### Radikalische Homopolymerisation von MA15-K5 in Lösung

Das Monomer MA15-K5 (0,05 mol/l) wurde in Toluol mit 2,2'-Azobisisobutyronitril (AIBN, 2,0 mol-%) bei 65 °C polymerisiert. Nach 15 h wurde die Polymerisation abgebrochen und das Polymerisat aus der 10-fachen Menge n-Hexan ausgefällt, abfiltriert und bis zur Gewichtskonstanz im Feinvakuum getrocknet. Es wurde ein weißes Polymer in 93 %iger Ausbeute erhalten. Die Polymerstruktur konnte mittels ¹H- und ¹³C-NMR-Spektroskopie bestätigt werden: 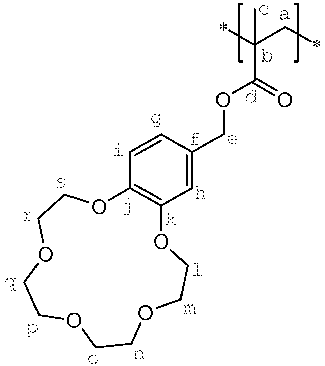 ¹H-NMR (400 MHz, CDCl₃, δ ppm) : 0,73-1,16 (H_{c}, m, 3H), 1,78-2,50 (Hₐ, m, 2H), 3,71 (Hₙ, Hₒ, Hₚ, H_{q}, s, 8H), 3,84 (Hₘ, Hᵣ, s, 4H), 4,07 (Hₗ, Hₛ, s, 4H), 4,80 (Hₑ, s, 2H), 6,80 (H_{g}, Hₕ, Hᵢ, m, 3H).
¹³C-NMR (100 MHz, CDCl₃, δ ppm): 16,7 + 18,9 C_{c}, 45,0 C_{b}, 66,8 C_{c}, 66,9-71,1 C₁-Cₛ, 113,7 Cₕ, 114,6 Cᵢ, 121,8 C_{g}, 128,3 C_{f}, 149,0 Cⱼ + Cₖ, 177,1 C_{d},

### Beispiel 6:

### Versetzende radikalische Polymerisation zur Ermittlung der Reaktivität in der radikalischen Polymerisation

In Schlenkgefäßen wurden homogene Mischungen aus jeweils in Tabelle 1 angegebenen Monomeren und Lösungsmitteln sowie 1 % AIBN hergestellt und mittels Durchleiten von Argon über eine Zeit von 20 min entgast. Danach wurden die Polymerisationsansätze in einem Thermostat auf 65 °C erwärmt. Die Zeit, bei der sich ein dreidimensionales standfestes Gel ausbildete, wurde als Gelzeit bestimmt.

**Tabelle 1: Zusammensetzung der Monomermischungen (Angaben in Gew.-%)**

| **Monomer** | **Lösungmittel** | **Gelzeit (min)** |
|---|---|---|
| 15 % MA-439 und 15 % GDMA | 69 % DMF | 15 |
| 15 % MA-442 und 15 % GDMA | 69 % Toluol | 5 |

Das Beispiel belegt eine deutlich größere radikalische Polymerisationsfähigkeit von MA-442, bei dem die polymerisationsfähige Gruppe über einen Spacer an dem N-heteroanalogen Kronenether-Ring angebunden ist, gegenüber MA-439, bei die Methacryloylgruppe direkt am Ring kovalent gebunden ist.

### Beispiel 7:

### Herstellung eines Dentinadhäsivs auf der Basis von MA15-K5

Zur Untersuchung der Dentinhaftung auf Rinderzahndentin wurde ein Adhäsiv mit der in Tabelle 2 angegebenen Zusammensetzung hergestellt:

**Tabelle 2: Zusammensetzung des Adhäsivs (Angaben in Gew.-%)**

| **Komponente** | **Gewichtsanteil** |
|---|---|
| MA15-K5 | 5,9 |
| EAEPA¹⁾ | 5,0 |
| Glycerindimethacrylat | 9,9 |
| UDMA²⁾ | 9,9 |
| Bis-GMA³⁾ | 32,7 |
| 2-Hydroxyethylmethacrylat | 14,9 |
| Photoinitiator⁴⁾ | 1,7 |
| Ethanol (abs.) | 20,0 |

| | |
|---|---|
| ¹⁾ EAEPA (2-[4-(Dihydroxyphosphoryl)-2-oxabutyl]-acrylsäure-ethylester), ²⁾ UDMA (Additionsprodukt aus 2-Hydroxyethylmethacrylat und 2,2,4-Trimethylhexamethylendiisocyanat), ³⁾ Bis-GMA (Additionsprodukt aus Methacrylsäure und Bisphenol-A-diglycidylether), ⁴⁾ Mischung aus Campherchinon (0,2 %), 4-Dimethylbenzoesäureethylester (0,5 %) und dem Acylphosphinoxid Lucirin TPO (1,0 %) | |

Rinderzähne wurden so in Kunststoffzylinder eingebettet, dass sich das Dentin und der Kunststoff in einer Ebene befanden. Nach 15 s Ätzung mit 37%iger Phosphorsäure wurde gründlich mit Wasser abgespült. Dann wurde mit einem Microbrush eine Schicht Adhäsiv vorstehender Zusammensetzung aufgepinselt, mit einem Luftgebläse zur Entfernung des Lösungsmittels kurz verblasen und für 40 s mit einer Halogenlampe (Astralis® 7, Ivoclar Vivadent AG) belichtet. Auf die Adhäsivschicht polymerisiert man einen Kompositzylinder aus Tetric^{®} Ceram (Ivoclar Vivadent AG) in zwei Schichten von je 1-2 mm auf. Anschließend wurden die Prüfkörper 24 h bei 37 °C in Wasser gelagert und die Scherhaftfestigkeit entsprechend der ISO-Richtlinie "ISO 2003-ISO TR 11405: Dental Materials Guidance on Testing of Adhesion to Tooth Structure" zu 14,8 MPa bestimmt, was einer guten Dentinhaftung entspricht.

### Beispiel 8:

### Herstellung eines Kompositzementes auf der Basis von MA15-K5

Entsprechend der nachfolgend aufgeführten Tabelle 3 wurden Kompositbefestigungszemente auf der Basis einer DimethacrylatMischung mit dem polymerisierbaren Kronenether MA15-K5 (Zement A), dem monofunktionellen Benzylmethacrylat (Zement B, Vergleichsbeispiel) und einer Mischung von MA15-K5 mit einem Phosphonsäuremonomer (Zement C) sowie jeweils einer Mischung aus Campherchinon und p-N,N-Dimethylaminobenzoesäureethylester als Photoinitiator mittels eines Walzenstuhles "Exakt" (Exakt Apparatebau, Norderstedt) hergestellt. Von den Materialien wurden entsprechende Prüfkörper präpariert, die 2 mal 3 min mit einer dentalen Lichtquelle (Spectramat^{®}, Ivoclar Vivadent AG) bestrahlt und ausgehärtet wurden. Nach der ISO-Norm ISO-4049 (Dentistry - Polymer-based filling, restorative and luting materials) erfolgte die Bestimmung der Biegefestigkeit und des Biege-E-Moduls.

**Tabelle 3: Zusammensetzung der aciden Kompositzemente (Angaben in Gew.-%)**

| **Komponente** | **Zement A** | **Zement B*** | **Zement C** |
|---|---|---|---|
| Campherchinon | 0,24 | 0,24 | 0,24 |
| p-N,N-Dimethylaminobenzoesäureethylester | 0,23 | 0,23 | 0,23 |
| UDMA¹⁾ | 27,75 | 27,75 | 23, 85 |
| Triethylenglycoldimethacrylat | 7,81 | 7,81 | 7,81 |
| Polymerisierbarer Kronenether MA15-K5 | 3, 90 | - | 3, 90 |
| Benzylmethacrylat | - | 3, 90 | - |
| Phosphonsäure MA-154²⁾ | | | 3, 90 |
| Aerosil® OX-50 (Evonik Degussa)³⁾ | 41,34 | 41,34 | 41,34 |
| Ytterbiumtrifluorid (Rhone-Poulenc)⁴⁾ | 18,73 | 18,73 | 18,73 |

| | | | |
|---|---|---|---|
| * Vergleichsbeispiel ¹⁾ Additionsprodukt aus 2 mol 2-Hydroxyethylmethacrylat und 1 mol 2,2,4-Trimethylhexamethylendiisocyanat ²⁾ 2-[2-Dihydroxyphosphoryl)-ethoxymethyl]-acrylsäureethylester ³⁾ silanisierte pyrogene Kieselsäure mit einer BET-Oberfläche von 50 ± 15 m²/g und einer mittleren Primärpartikelgröße von 40 nm ⁴⁾ Partikelgröße von 200 bis 250 nm | | | |

**Tabelle 4: Kompositzementeigenschaften**

| **Materialeigenschaft** | **Zement A** | **Zement B*** | **Zement C** |
|---|---|---|---|
| Biegefestigkeit (MPa) nach 24 h WL¹⁾ | 109 | 109 | 111 |
| E-Modul (MPa) nach 24 h WL¹⁾ | 5165 | 5200 | 5100 |

| | | | |
|---|---|---|---|
| * Vergleichsbeispiel ¹⁾ WL = Wasserlagerung der Prüfkörper bei 37 °C | | | |

Aus Tabelle 4 ist ersichtlich, dass der auf polymerisierbaren Kronenether MA15-K5 basierende Zement A bzw. auf der Mischung von MA15-K5 und einem Phosphonsäuremonomer basierende Zement C, im Vergleich zum Zement B (konventioneller Zement ohne Kronenether und Phoshonsäure) zu vergleichbaren mechanischen Eigenschaften führen und damit keine Beeinträchtigung durch das polarere Kronenethermonomer auftritt.

### Beispiel 9:

### Herstellung eines Komposits auf der Basis von MA15-K5

Entsprechend der nachfolgend aufgeführten Tabelle 5 wurden Komposite auf der Basis einer Dimethacrylatmischung und unter Einbeziehung des polymerisierbaren Kronenether MA15-K5 (Komposit A) oder dem monofunktionellen Benzylmethacrylat (Komposit B, Vergleichsbeispiel) sowie einer Mischung aus Campherchinon (CC) und p-N,N-Dimethylaminobenzoesäureethylester (EMBO) als Photoinitiator mittels eines Kneters LPM 0.1 SP (Linden, Marienheide) hergestellt. Von den Materialien wurden entsprechende Prüfkörper präpariert, die 2 mal 3 Minuten mit einer dentalen Lichtquelle (Spectramat^{®}, Ivoclar Vivadent AG) bestrahlt und damit ausgehärtet wurden. Nach der ISO-Norm ISO-4049 (Dentistry - Polymer-based filling, restorative and luting materials) erfolgte die Bestimmung der Biegefestigkeit und des Biege-E-Moduls (Tabelle 6).

**Tabelle 5: Zusammensetzung der Füllungskomposite (Angaben in Gew.-%)**

| **Komponente** | **Komposit A** | **Komposit B*** |
|---|---|---|
| Monomerharz¹⁾ | 18,11 | 18,11 |
| Polymerisierbarer Kronenether MA15-K5 | ja | - |
| Benzylmethacrylat | - | ja |
| Glasfüller GM27884 (Schott)²⁾ | 51, 61 | 51, 61 |
| Sphärosil (Tokoyama Soda)³⁾ | 14,39 | 14,39 |
| Ytterbiumtrifluorid (Rhone-Poulenc)⁴⁾ | 14,89 | 14,89 |
| Aerosil® OX-50 (Evonik Degussa)⁵⁾ | 1,00 | 1,00 |

| | | |
|---|---|---|
| * Vergleichsbeispiel ¹⁾ Mischung aus 38,00 Gew.-% Bis-GMA, 34,12 Gew.-% UDMA, 17,07 Gew.-% Triethylenglycoldimethacrylat, 0,31 Gew.-% CC, 0,50 Gew.% EMBO und jeweils 10,00 Gew.-% MA15-K5 (Komposit A) oder Benzylmethacrylat (Komposit B) ²⁾ Silanisierter Ba-Al-Borosilicatglasfüller mit einer mittleren Partikelgröße von 1,5 µm, ³⁾ SiO₂-ZrO₂-Mischoxid mit einer mittlere Primärpartikelgröße von 250 nm ⁴⁾ Partikelgröße von 200 bis 250 nm ⁵⁾ silanisierte pyrogene Kieselsäure mit einer BET-Oberfläche von 50 ± 15 m²/g und einer mittleren Primärpartikelgröße von 40 nm | | |

**Tabelle 6: Füllungskompositeigenschaften**

| **Materialeigenschaft** | **Komposit A** | **Komposit B*** |
|---|---|---|
| Biegefestigkeit (MPa) nach 24 h WL¹⁾ | 162 | 159 |
| Biege-E-Modul (GPa) nach 24 h WL¹⁾ | 13170 | 13890 |

| | | |
|---|---|---|
| * Vergleichsbeispiel ¹⁾ WL = Wasserlagerung der Prüfkörper bei 37 °C | | |

Aus Tabelle 6 ist ersichtlich, dass das auf dem polymerisierbaren Kronenether MA15-K5 basierende Komposit A im Vergleich zum Komposit B (konventionelles Komposit ohne Kronenether) zu vergleichbaren mechanischen Eigenschaften führt und damit keine Beeinträchtigung der üblichen Kompositeigenschaften durch das polarere Kronenethermonomer auftritt.

## Patentansprüche

1. Dentalmaterial enthaltend mindestens 0,05 Gew.-%, bezogen auf das Gesamtgewicht des Dentalmaterials, mindestens eines radikalisch polymerisierbaren makrocyclischen Polyethers oder radikalisch polymerisierbaren makrocyclischen heteroanalogen Polyethers.

2. Dentalmaterial nach Anspruch 1, bei dem der radikalisch polymerisierbare makrocyclische Polyether oder makrocyclische heteroanaloge Polyether der allgemeinen Formel I MC-(SP-PG)ₙ entspricht, worin
MC einem n-fach substituierten Rest
(a) eines Kronenethers oder heteroanalogen Kronenethers der allgemeinen Formel IIa 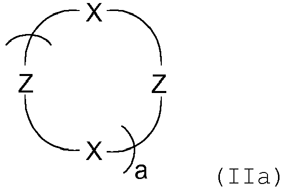 oder
(b) eines Kryptanden der Formel IIb 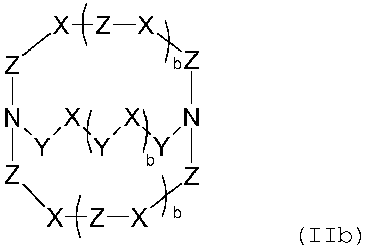 entspricht, worin
X unabhängig voneinander jeweils aus O, S und NR¹ ausgewählt ist, wobei R¹ unabhängig voneinander für H, den Rest SP-PG oder einen anderen organischen Rest steht;
Y unabhängig voneinander aus C₂-C₄-Alkylen-Resten ausgewählt ist,
Z unabhängig voneinander jeweils aus C₁-C₄-Alkylen-, C₆-C₁₀-Arylen- und C₄-C₈-Cycloalkylen-Resten ausgewählt ist;
a eine ganze Zahl von 3 bis 10 ist und
b unabhängig voneinander eine ganze Zahl von 1 bis 3 ist;
SP unabhängig voneinander eine Verbindungsgruppe ist oder entfällt,
PG eine radikalisch polymerisierbare Gruppe ist, und
n eine ganze Zahl von 1 bis 8 ist,
wobei SP-PG und/oder PG an MC über mindestens einen der Z-Reste und für X = NR¹ zusätzlich oder ausschließlich über mindestens ein N-Atom gebunden ist.

3. Dentalmaterial nach einem der vorstehenden Ansprüche, bei dem MC der Formel IIa entspricht und die X-Gruppen O-Atome oder eine Kombination von O-Atomen und S-Atomen sind.

4. Dentalmaterial nach Anspruch 3, bei dem (a+1-n) Z-Reste Ethylen-Reste sind und n Z-Reste PG- und/oder SP-PG-substituierte C₆-C₁₀-Arylen- und/oder C₄-C₈-Cycloalkylen-Reste, vorzugsweise PG- und/oder SP-PG-substituierte Phenylen- und/oder Cyclohexylen-Reste sind.

5. Dentalmaterial nach einem der Ansprüche 1 oder 2, bei dem MC der Formel IIa entspricht und die X-Gruppen NR¹-Gruppen oder eine Kombination von O-Atomen und NR¹-Gruppen sind.

6. Dentalmaterial nach Anspruch 5, bei dem R¹ in n NR¹-Gruppen einem PG- und/oder SP-PG-Rest entspricht und alle Z-Reste Ethylen-Reste sind.

7. Dentalmaterial nach einem der Ansprüche 1 oder 2, bei dem MC der Formel IIb entspricht, die X-Gruppen O-Atome sind, die Y-Reste Ethylen-Reste sind, (2b+2-n) Z-Reste Ethylen-Reste sind und n Z-Reste PG- und/oder SP-PG-substituierte C₁-C₄-Alkylen-, C₆-C₁₀-Arylen- und/oder C₄-C₈-Cycloalkylen-Reste, vorzugsweise PG- und/oder SP-PG-substituierte Phenylen- und/oder Ethylen-Reste sind.

8. Dentalmaterial nach einem der vorstehenden Ansprüche, bei dem die radikalisch polymerisierbare Gruppe(n) PG Vinyl-, Allyl- und/oder (Meth)acryloylgruppen ist (sind).

9. Dentalmaterial nach einem der vorstehenden Ansprüche, bei dem die Verbindungsgruppe (n) SP der Formel -R⁴-Z¹-R⁵-Z²-entspricht (entsprechen), worin Z¹ und Z² gleich oder unterschiedlich sind und jeweils für -O-, -CO-O-, -O-CO-, - CO-NH-, -NH-CO-, -O-CO-NH- oder -NH-CO-O- stehen oder eines oder beide von Z¹ und Z² entfallen und R⁴ und R⁵ gleich oder unterschiedlich sind und jeweils für einen C₁-C₁₀-Alkylenrest stehen oder eines oder beide von R⁴ und R⁵ entfallen.

10. Dentalmaterial nach einem der vorstehenden Ansprüche, das ein Adhäsiv, ein Beschichtungsmaterial, ein Zement oder ein Komposit ist.

11. Dentalmaterial nach Anspruch 10, das ein Adhäsiv oder ein Beschichtungsmaterial ist und enthält:
(i) 0,05 bis 40 Gew.-%, bevorzugt 1 bis 30 Gew.-% und besonders bevorzugt 1 bis 20 Gew.-% des mindestens einen radikalisch polymerisierbaren makrocyclischen Polyethers und/oder makrocyclischen heteroanalogen Polyethers,
(ii) 0,01 bis 10 Gew.-%, bevorzugt 0,1 bis 3,0 Gew.-% eines Initiators für die radikalische Polymerisation,
(iii) 0 bis 80 Gew.-%, bevorzugt 0 bis 60 Gew.-% und besonders bevorzugt 5 bis 50 Gew.-% mindestens eines radikalisch polymerisierbaren Monomers, das sich von Komponente (i) unterscheidet,
(iv) 0 bis 20 Gew.-% Füllstoff und
(v) 0 bis 95 Gew.-%, bevorzugt 0 bis 70 Gew.-% und besonders bevorzugt 5 bis 50 Gew.-% Lösungsmittel,
jeweils bezogen auf das Gesamtgewicht des Dentalmaterials.

12. Dentalmaterial nach Anspruch 10, das ein Zement oder ein Komposit ist und enthält:
(i) 0,05 bis 40 Gew.-%, bevorzugt 1 bis 30 Gew.-% und besonders bevorzugt 1 bis 20 Gew.-% des mindestens einen radikalisch polymerisierbaren makrocyclischen Polyethers und/oder makrocyclischen heteroanalogen Polyethers,
(ii) 0,01 bis 10 Gew.-%, bevorzugt 0,1 bis 3,0 Gew.-% eines Initiators für die radikalische Polymerisation,
(iii) 0 bis 80 Gew.-%, bevorzugt 0 bis 60 Gew.-% und besonders bevorzugt 5 bis 50 Gew.-% mindestens eines radikalisch polymerisierbaren Monomers, das sich von Komponente (i) unterscheidet, und
(iv) 20 bis 75 Gew.-% Füllstoff,
jeweils bezogen auf das Gesamtgewicht des Dentalmaterials.

13. Verwendung eines radikalisch polymerisierbaren makrocyclischen Polyethers oder makrocyclischen heteroanalogen Polyethers wie in einem der Ansprüche 1 bis 10 definiert zur Herstellung eines Dentalmaterials.

14. Radikalisch polymerisierbarer makrocyclischer Polyether oder makrocyclischer heteroanaloger Polyether wie in einem der Ansprüche 1 bis 10 definiert zur Verwendung in der Zahnheilkunde.

## Claims

1. Dental material comprising at least 0.05 wt %, relative to the total weight of the dental material, of at least one radically polymerisable macrocyclic polyether or radically polymerisable macrocyclic heteroanalogous polyether.

2. Dental material according to claim 1, **characterised in that** the radically polymerisable macrocyclic polyether or macrocyclic heteroanalogous polyether corresponds to the general Formula I MC-(SP-PG)ₙ, in which
MC corresponds to an n-times substituted residue
(a) of a crown ether or heteroanalogous crown ether of the general Formula IIa 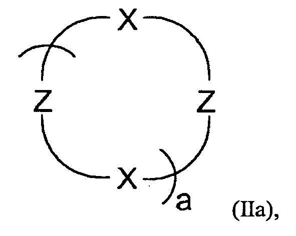 or
(b) of a cryptand of the Formula IIb 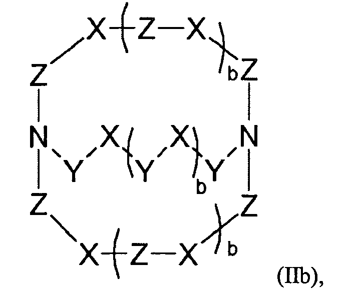 in which
X is selected independently of each other in each case from O, S and NR¹, wherein R¹ stands independently of each other for H, the residue SP-PG or another organic group;
Y is selected independently of each other from C₂-C₄ alkylene groups,
Z is selected independently of each other in each case from C₁-C₄ alkylene, C₆-C₁₀ arylene and C₄-C₈ cycloalkylene groups;
a is a whole number from 3 to 10 and
b independently of each other is a whole number from 1 to 3;
SP independently of each other is a linking group or is absent,
PG is a radically polymerisable group and
n is a whole number from 1 to 8,
wherein SP-PG and/or PG is bonded to MC through at least one of the Z groups, and additionally or exclusively through at least one N atom when X = NR¹.

3. Dental material according to one of the preceding claims, **characterised in that** MC conforms to the Formula IIa and the X groups are O atoms or a combination of O atoms and S atoms.

4. Dental material according to claim 3, **characterised in that** (a+1-n) Z groups are ethylene groups and n Z groups are PG- and/or SP-PG-substituted C₆-C₁₀ arylene and/or C₄-C₈ cycloalkylene groups, preferably PG- and/or SP-PG-substituted phenylene and/or cyclohexylene groups.

5. Dental material according to one of claims 1 or 2, **characterised in that** MC conforms to the Formula IIa and the X groups are NR¹ groups or a combination of O atoms and NR¹ groups.

6. Dental material according to claim 5, **characterised in that** R¹ in n NR¹ groups corresponds to a PG and/or SP-PG residue and all Z groups are ethylene groups.

7. Dental material according to one of claims 1 or 2, **characterised in that** MC conforms to the Formula IIb, the X groups are O atoms, the Y groups are ethylene groups, (2b+2-n) Z groups are ethylene groups and n Z groups are PG- and/or SP-PG-substituted C₁-C₄ alkylene, C₆-C₁₀ arylene and/or C₄-C₈ cycloalkylene groups, preferably PG- and/or SP-PG-substituted phenylene and/or ethylene groups.

8. Dental material according to one of the preceding claims, **characterised in that** the radically polymerisable group(s) PG is (are) vinyl, allyl and/or (meth)acryloyl groups.

9. Dental material according to one of the preceding claims, **characterised in that** the linking group(s) SP conforms (conform) to the Formula -R⁴-Z¹-R⁵-Z²-, in which Z¹ and Z² are the same or different and in each case stand for -O-, -CO-O-, -O-CO-, -CO-NH-, -NH-CO-, -O-CO-NH- or-NH-CO-O- or one or both of Z¹ and Z² are absent and R⁴ and R⁵ are the same or different and in each case stand for a C₁-C₁₀ alkylene group or one or both of R⁴ and R⁵ are absent.

10. Dental material according to one of the preceding claims, **characterised in that** it is an adhesive, a coating material, a cement or a composite.

11. Dental material according to claim 10, **characterised in that** it is an adhesive or a coating material and comprises:
(i) 0.05 to 40 wt %, preferably 1 to 30 wt % and particularly preferably 1 to 20 wt % of the at least one radically polymerisable macrocyclic polyether and/or macrocyclic heteroanalogous polyether,
(ii) 0.01 to 10 wt %, particularly preferably 0.1 to 3.0 wt %, of an initiator for the radical polymerisation,
(iii) 0 to 80 wt %, preferably 0 to 60 wt %, and particularly preferably 5 to 50 wt % of at least one radically polymerisable monomer that is different from component (i),
(iv) 0 to 20 wt % filler and
(v) 0 to 95 wt %, preferably 0 to 70 wt %, and particularly preferably 5 to 50 wt % solvent,
each relative to the total weight of the dental material.

12. Dental material according to claim 10, **characterised in that** it is an adhesive or a coating material and comprises:
(i) 0.05 to 40 wt %, preferably 1 to 30 wt % and particularly preferably 1 to 20 wt % of the at least one radically polymerisable macrocyclic polyether and/or macrocyclic heteroanalogous polyether,
(ii) 0.01 to 10 wt %, particularly preferably 0.1 to 3.0 wt % of an initiator for the radical polymerisation,
(iii) 0 to 80 wt %, preferably 0 to 60 wt % and particularly preferably 5 to 50 wt % of at least one radically polymerisable monomer that is different from component (i), and
(iv) 20 to 75 wt % filler,
each relative to the total weight of the dental material.

13. Use of a radically polymerisable macrocyclic polyether or macrocyclic heteroanalogous polyether, as defined in one of claims 1 to 10, for the preparation of a dental material.

14. Radically polymerisable macrocyclic polyether or macrocyclic heteroanalogous polyether, as defined in one of claims 1 to 10, for use in restorative dentistry.

## Revendications

1. Matériau dentaire contenant au moins 0,05 %, en poids rapporté au poids total du matériau dentaire, d'au moins un polyéther macrocyclique polymérisable par voie radicalaire ou hétéro-analogue de polyéther macrocyclique polymérisable par voie radicalaire.

2. Matériau dentaire conforme à la revendication 1, dans lequel le polyéther macrocyclique ou hétéro-analogue de polyéther macrocyclique, polymérisable par voie radicalaire, correspond à la formule générale
MC-(SP-PG)ₙ (I)
dans laquelle
- MC représente le reste, porteur de n substituant(s),
a) d'un éther-couronne ou hétéro-analogue d'éther-couronne, de formule générale IIa : 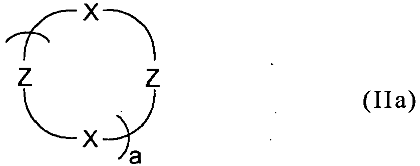
b) ou d'un cryptand, de formule générale IIb : 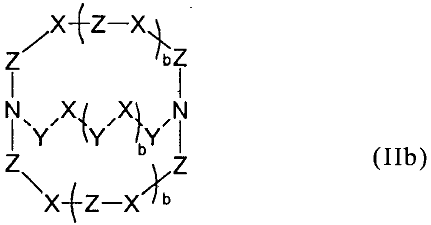 dans lesquelles formules
- les symboles X représentent des chaînons choisis, indépendamment les uns des autres, parmi ceux symbolisés par O, S ou NR¹, où les symboles R¹, indépendamment les uns des autres, représentent chacun un atome d'hydrogène, un reste symbolisé par SP-PG, ou un autre reste organique,
- les symboles Y représentent des entités choisies, indépendamment les unes des autres, parmi les groupes alcanediyle en C₂-C₄,
- les symboles Z représentent des entités choisies, indépendamment les unes des autres, parmi les groupes alcanediyle en C₁-C₄, arènediyle en C₆-C₁₀, et cycloalcanediyle en C₄-C₈,
- l'indice a est un nombre entier qui vaut de 3 à 10,
- et les indices b sont chacun, indépendamment les uns des autres, un nombre entier qui vaut de 1 à 3 ;
- les symboles SP, indépendamment les uns des autres, représentent un groupe de raccordement ou ne représentent rien ;
- PG représente un groupe polymérisable par voie radicalaire ;
- et l'indice n est un nombre entier qui vaut de 1 à 8 ;
étant entendu que le groupe symbolisé par SP-PG et/ou PG est lié au groupe symbolisé par MC par l'intermédiaire d'au moins l'un des groupes symbolisés par Z, et dans le cas où X représente un chaînon symbolisé par NR¹, par l'intermédiaire, en plus ou exclusivement, d'au moins un atome d'azote.

3. Matériau dentaire conforme à l'une des revendications précédentes, dans lequel MC représente un reste de formule IIa et les entités symbolisées par X sont des atomes d'oxygène ou une combinaison d'atomes d'oxygène et d'atomes de soufre.

4. Matériau dentaire conforme à la revendication 3, dans lequel a+1-n restes symbolisés par Z sont des groupes éthanediyle et n restes symbolisés par Z sont des groupes arènediyle en C₆-C₁₀ et/ou cycloalcanediyle en C₄-C₈, porteurs de substituant(s) symbolisé(s) par PG et/ou SP-PG, de préférence des groupes phénylène et/ou cyclohexanediyle, porteurs de substituant(s) symbolisé(s) par PG et/ou SP-PG.

5. Matériau dentaire conforme à l'une des revendications 1 et 2, dans lequel MC représente un reste de formule IIa et les entités symbolisées par X sont des chaînons symbolisés par NR¹, ou une combinaison d'atomes d'oxygène et de chaînons symbolisés par NR¹.

6. Matériau dentaire conforme à la revendication 5, dans lequel, dans les n chaînons symbolisés par NR¹, R¹ représente un reste symbolisé par PG et/ou SP-PG, et tous les restes symbolisés par Z sont des groupes éthanediyle.

7. Matériau dentaire conforme à l'une des revendications 1 et 2, dans lequel MC représente un reste de formule IIb et les entités symbolisées par X sont des atomes d'oxygène, les entités symbolisées par Y sont des groupes éthanediyle, 2b+2-n restes symbolisés par Z sont des groupes éthanediyle, et n restes symbolisés par Z sont des groupes alcanediyle en C₁-C₄, arènediyle en C₆-C₁₀ et/ou cycloalcanediyle en C₄-C₈, porteurs de substituant(s) symbolisé(s) par PG et/ou SP-PG, de préférence des groupes phénylène et/ou éthanediyle, porteurs de substituant(s) symbolisé(s) par PG et/ou SP-PG.

8. Matériau dentaire conforme à l'une des revendications précédentes, dans lequel le ou les groupe(s) polymérisable(s) par voie radicalaire, symbolisé(s) par PG, est ou sont un ou des groupes vinyle, allyle et/ou acryloyle ou méthacryloyle.

9. Matériau dentaire conforme à l'une des revendications précédentes, dans lequel le ou les groupe(s) de raccordement, symbolisé(s) par SP, est ou sont de formule -R⁴-Z¹-R⁵-Z²-, dans laquelle Z¹ et Z² représentent des entités identiques ou différentes qui sont chacune un raccord symbolisé par -O-, -CO-O-, -O-CO-, -CO-NH-, -NH-CO-, -O-CO-NH- ou -NH-CO-O-, ou l'un de ces symboles Z¹ et Z² ou les deux ne représente(nt) rien, et R⁴ et R⁵ représentent des entités identiques ou différentes qui sont chacune un groupe alcanediyle en C₁-C₁₀, ou l'un de ces symboles R⁴ et R⁵ ou les deux ne représente(nt) rien.

10. Matériau dentaire conforme à l'une des revendications précédentes, qui est un adhésif, un matériau d'enduction, un ciment ou un composite.

11. Matériau dentaire conforme à la revendication 10, qui est un adhésif ou un matériau d'enduction et qui contient :
i) de 0,05 à 40 % en poids, de préférence de 1 à 30 % en poids, et en particulier de 1 à 20 % en poids, d'au moins un polyéther macrocyclique et/ou hétéroanalogue de polyéther macrocyclique, polymérisable par voie radicalaire,
ii) de 0,01 à 10 % en poids, et de préférence de 0,1 à 3,0 % en poids, d'un amorceur de polymérisation par voie radicalaire,
iii) de 0 à 80 % en poids, de préférence de 0 à 60 % en poids, et en particulier de 5 à 50 % en poids, d'au moins un monomère polymérisable par voie radicalaire, distinct du composant (i),
iv) de 0 à 20 % en poids d'une charge,
v) et de 0 à 95 % en poids, de préférence de 0 à 70 % en poids, et en particulier de 5 à 50 % en poids, d'un solvant,
chacun de ces pourcentages étant rapporté au poids total du matériau dentaire.

12. Matériau dentaire conforme à la revendication 10, qui est un ciment ou un composite et qui contient :
i) de 0,05 à 40 % en poids, de préférence de 1 à 30 % en poids, et en particulier de 1 à 20 % en poids, d'au moins un polyéther macrocyclique et/ou hétéroanalogue de polyéther macrocyclique, polymérisable par voie radicalaire,
ii) de 0,01 à 10 % en poids, et de préférence de 0,1 à 3,0 % en poids, d'un amorceur de polymérisation par voie radicalaire,
iii) de 0 à 80 % en poids, de préférence de 0 à 60 % en poids, et en particulier de 5 à 50 % en poids, d'au moins un monomère polymérisable par voie radicalaire, distinct du composant (i),
iv) et de 20 à 75 % en poids d'une charge,
chacun de ces pourcentages étant rapporté au poids total du matériau dentaire.

13. Utilisation d'un polyéther macrocyclique ou hétéroanalogue de polyéther macrocyclique, polymérisable par voie radicalaire, tel que défini dans l'une des revendications 1 à 10, pour la préparation d'un matériau dentaire.

14. Polyéther macrocyclique ou hétéroanalogue de polyéther macrocyclique, polymérisable par voie radicalaire, tel que défini dans l'une des revendications 1 à 10, pour utilisation en chirurgie dentaire.
